(19) Europäisches Patentamt
European Patent Office
Office européen des brevets

(11) **EP 4 542 486 A1**

(12) **EUROPEAN PATENT APPLICATION**
published in accordance with Art. 153(4) EPC

(43) Date of publication:
**23.04.2025 Bulletin 2025/17**

(21) Application number: 23830390.3

(22) Date of filing: **29.06.2023**

(51) International Patent Classification (IPC):
*G06T 7/11* $^{(2017.01)}$

(52) Cooperative Patent Classification (CPC):
G06T 7/11

(86) International application number:
**PCT/CN2023/103728**

(87) International publication number:
**WO 2024/002221 (04.01.2024 Gazette 2024/01)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 30.06.2022  CN 202210761324
29.07.2022  CN 202210907258

(71) Applicant: **Wuhan United Imaging Surgical Co.,
Ltd.**
**Wuhan, Hubei 430073 (CN)**

(72) Inventors:
• LIAO, Mingzhe
  Wuhan, Hubei 430073 (CN)
• ZHANG, Tian
  Wuhan, Hubei 430206 (CN)
• FANG, Wei
  Wuhan, Hubei 430206 (CN)
• ZHANG, Jing
  Wuhan, Hubei 430206 (CN)

(74) Representative: **Wang, Bo**
**Panovision IP**
**Ebersberger Straße 3**
**85570 Markt Schwaben (DE)**

(54) **IMAGING-ASSISTED METHOD, SYSTEM AND APPARATUS FOR INTERVENTIONAL
OPERATION, AND STORAGE MEDIUM**

(57)    The embodiments of the present disclosure provide a method for an image-assisted interventional surgery. The method comprises: obtaining a medical image; segmenting a target structure set from the medical image; and determining a result structure set relating to one or more non-interventional regions based on a segmentation result of the target structure set.

**200A**

FIG. 2A

## Description

### CROSS-REFERENCE TO RELATED APPLICATIONS

This application claims priority to the Chinese Patent Application No. 2022107613241, filed on June 30, 2022, and the Chinese Patent Application No. 202210907258.4, filed on July 29, 2022, the entire contents of each of which are incorporated herein by reference.

### TECHNICAL FIELD

The present disclosure relates to the field of image processing technology, and in particular to methods, systems, devices, and storage media for an image-assisted interventional surgery.

### BACKGROUND

Pre-operative planning for an interventional surgery (hereinafter referred to as pre-operative planning) refers to making a surgical plan by acquiring images of the blood vessels, lesions, organs, etc., of a scanned subject (e.g., a patient, etc.) with the assistance of a medical scanning device to reasonably avoid blood vessels and important organs in combination with pathological and anatomical knowledge, and introducing special precision instruments into the lesions. Whether the pre-operative planning can accurately determine the spatial positions of the blood vessels and the lesions in medical scan images, and reasonably plan a puncture path of a puncture needle directly affects whether the organs and the blood vessels can be effectively avoided during the surgery to make the puncture needle reach the lesions smoothly. Therefore, it is particularly important to provide a solution for an image-assisted interventional surgery, which makes the pre-operative planning achieve a higher accuracy to better assist in the accurate implementation of the corresponding puncture path during the operation, thereby obtaining the ideal surgical effect.

### SUMMARY

One of the embodiments of the present disclosure provides a method for an image-assisted interventional surgery. The method may include: obtaining a medical image; segmenting a target structure set from the medical image; and determining a result structure set relating to one or more non-interventional regions based on a segmentation result of the target structure set.

In some embodiments, the medical image may include a pre-operative image with contrast and an intra-operative image without contrast. The target structure set may include a first target structure set of the pre-operative image with contrast and a second target structure set of the intra-operative image without contrast. The result structure set may include a third intra-operative target structure set. The segmenting a target structure set from the medical image may include: obtaining a first segmentation image of the first target structure set by segmenting the first target structure set from the pre-operative image with contrast; and obtaining a second segmentation image of the second target structure set by segmenting the second target structure set from the intra-operative image without contrast; the first target structure set and the second target structure set having an intersection. The determining a result structure set relating to one or more non-interventional regions based on a segmentation result of the target structure set may include: determining a spatial position of the third intra-operative target structure set by registering the first segmentation image with the second segmentation image; at least one element of the third target structure set being included in the first target structure set, and at least one element of the third target structure set being not included in the second target structure set.

In some embodiments, the method for the image-assisted interventional surgery may further include: obtaining a planning mode of the interventional surgery, the planning mode including a fast segmentation mode and/or a precise segmentation mode; and segmenting a fourth target structure set from the intra-operative image without contrast according to the planning mode.

In some embodiments, in the fast segmentation mode, the fourth target structure set may include the one or more non-interventional regions.

In some embodiments, in the precise segmentation mode, the fourth target structure set may include one or more preset important organs.

In some embodiments, a ratio of a total volume of the one or more preset important organs to a total volume of the one or more non-interventional regions of the fourth target structure set may be less than a preset efficiency factor m.

In some embodiments, the preset efficiency factor m may be set based on a type of the interventional surgery.

In some embodiments, the segmentation may include: performing a coarse segmentation on at least one element of the target structure set in the medical image; obtaining a mask of the at least one element; determining positioning information of the mask; and performing a precise segmentation on the at least one element based on the positioning information of the mask.

In some embodiments, the registering the first segmentation image with the second segmentation image may include: determining a registration deformation field by registering the first segmentation image with the second segmentation image; and determining, based on the registration deformation field and a spatial position of at least part of the at least one element of the first target structure set in the pre-operative image with contrast, a spatial position of the at least one element during the surgery.

In some embodiments, the determining a registration deformation field may include: determining a first preliminary deformation field based on a registration result corresponding to the at least one element; determining a second preliminary deformation field of a full image based on the first preliminary deformation field, the full image including the at least part of the at least one element; determining a registration map of a moving image by deforming the moving image based on the second preliminary deformation field; obtaining a third preliminary deformation field by registering a region within a first grayscale difference range in the registration map of the moving image with a region within the first grayscale difference range of a fixed image; determining a fourth preliminary deformation field of the full image based on the third preliminary deformation field; and obtaining a final registration map by registering a region of the registration map based on the fourth preliminary deformation field, the region being within a second grayscale difference range with respect to the fixed image.

In some embodiments, the result structure set may include a fat-free mask. The segmenting a target structure set from the medical image may include: obtaining the fat-free mask by segmenting the target structure set from the medical image. The determining a result structure set relating to one or more non-interventional regions based on a segmentation result of the target structure set may include: determining a first intersection between a target organ mask and the fat-free mask within a first preset range, and adjusting regions of the target organ mask and the fat-free mask based on the first intersection to obtain an adjusted fat-free mask.

In some embodiments, the method for the image-assisted interventional surgery may further include: performing a connected domain processing on the adjusted fat-free mask; and obtaining a processed fat-free mask based on a fat-free mask after the connected domain processing.

In some embodiments, the determining a first intersection between a target organ mask and the fat-free mask within a preset range, and adjusting regions of the target organ mask and the fat-free mask based on the first intersection may include: detecting the target organ mask; determining the first intersection between the target organ mask and the fat-free mask within the first preset range based on a detection result, wherein the first preset range may be determined according to a first preset parameter; and performing a first adjustment on the regions of the target organ mask and the fat-free mask based on the first intersection.

In some embodiments, the method may further include: determining a second intersection between the target organ mask after the first adjustment and the fat-free mask after the first adjustment within a second preset range, wherein the second preset range may be determined according to a second preset parameter; and performing a second adjustment on the regions of the target organ mask after the first adjustment and the fat-free mask after the first adjustment based on the second intersection.

In some embodiments, the second preset parameter may be less than or equal to the first preset parameter. At least one of the first preset parameter or the second preset parameter may be obtained based on big data or artificial intelligence (AI).

In some embodiments, the performing a connected domain processing on the adjusted fat-free mask may include: determining whether positioning information of the target organ mask overlaps with positioning information of a pseudo fat-free connected domain; in response to determining that the positioning information of the target organ mask does not overlap with the positioning information of the pseudo fat-free connected domain, determining that the pseudo fat-free connected domain belongs to the fat-free mask; or in response to determining that the positioning information of the target organ mask overlaps with the positioning information of the pseudo fat-free connected domain, determining whether the pseudo fat-free connected domain belongs to the fat-free mask according to a relationship between an area of the pseudo fat-free connected domain and a preset area threshold.

In some embodiments, the determining whether the pseudo fat-free connected domain belongs to the fat-free mask according to a relationship between an area of the pseudo fat-free connected domain and a preset area threshold may include: in response to determining that the area of the pseudo fat-free connected domain is greater than the preset area threshold, determining that the pseudo fat-free connected domain belongs to the fat-free mask; or in response to determining that the area of the pseudo fat-free connected domain is less than or equal to the preset area threshold, determining that the pseudo fat-free connected domain does not belong to the fat-free mask.

In some embodiments, the method may further include: making the pseudo fat-free connected domain with at least one of a retaining identifier or a discarding identifier. The retaining identifier represents pseudo fat-free connected domains belonging to the fat-free mask. The discarding identifier represents pseudo fat-free connected domains not belonging to the fat-free mask.

In some embodiments, the obtaining a processed fat-free mask based on the fat-free mask after the connected domain processing may include: detecting the adjusted target organ mask; determining an adjacent boundary of the fat-free mask after the connected domain processing and the target organ mask based on a detection result; and obtaining the processed fat-free mask by performing a third adjustment on the fat-free mask after the connected domain processing based on the adjacent boundary.

In some embodiments, the method may further include: obtaining an operation instruction; obtaining at least one target organ mask by segmenting at least one target organ from the medical image according to the operation instruction; determining a third intersection between the at least one target organ mask and a fast segmentation result mask within a first preset range, and adjusting regions of the at least one target organ mask and the fast segmentation result mask based on the third intersection, wherein the fast segmentation result mask may include at least the processed fat-free mask; performing a connected domain processing on the adjusted fast segmentation result mask; and obtaining a processed fast segmentation result mask based on the fast segmentation result mask after the connected domain processing.

One of the embodiments of the present disclosure provides a system for an image-assisted interventional surgery. The system may include an obtaining module configured to obtain a medical image; a segmentation module configured to segment a target structure set from the medical image; and a result determination module configured to determine a result structure set relating to one or more non-interventional regions based on a segmentation result of the target structure set.

One of the embodiments of the present disclosure provides a non-transitory computer-readable storage medium, comprising computer instructions that, when read by a computer, may direct the computer to implement the method for the image-assisted interventional surgery of any embodiment of the present disclosure.

One of the embodiments of the present disclosure provides a device for an image-assisted interventional surgery. The device may include a processor. The processor may be configured to implement the method for the image-assisted interventional surgery of any embodiment of the present disclosure.

In some embodiments, the device for the image-assisted interventional surgery may further include a display device. The display device may be configured to display a segmentation result of the method for the image-assisted interventional surgery implemented by the processor. The display device may be further configured to display trigger mode options. The trigger mode options may include a fast segmentation mode and/or a precise segmentation mode.

**BRIEF DESCRIPTION OF THE DRAWINGS**

The present disclosure will be further illustrated by way of exemplary embodiments, which will be described in detail by means of the accompanying drawings. These embodiments are not limiting, and in these embodiments, the same numbering indicates the same structure, wherein:

FIG. 1 is a schematic diagram illustrating an application scenario of a system for an image-assisted interventional surgery according to some embodiments of the present disclosure;
FIG. 2A is a flowchart illustrating an exemplary method for an image-assisted interventional surgery according to some embodiments of the present disclosure;
FIG. 2B is a flowchart illustrating an exemplary method for an image-assisted interventional surgery according to some embodiments of the present disclosure;
FIG. 3 is a flowchart illustrating an exemplary segmentation process in a method for an image-assisted interventional surgery according to some embodiments of the present disclosure;
FIG. 4 is a flowchart illustrating an exemplary process of determining positioning information of an element mask according to some embodiments of the present disclosure;
FIG. 5 is a flowchart illustrating an exemplary process of performing soft connected domain analysis on an element mask according to some embodiments of the present disclosure;
FIG. 6 is a comparison diagram illustrating exemplary coarse segmentation effects of performing soft connected domain analysis on an element mask according to some embodiments of the present disclosure;
FIG. 7 is a flowchart illustrating an exemplary process of precise segmentation on at least one element according to some embodiments of the present disclosure;
FIG. 8 is a schematic diagram illustrating an exemplary process of determining positioning information of an element mask according to some embodiments of the present disclosure;
FIG. 9 is a schematic diagram illustrating an exemplary process of determining positioning information of an element mask according to some embodiments of the present disclosure;
FIG. 10A is a schematic diagram illustrating an exemplary process of determining a sliding direction based on positioning information of an element mask according to some embodiments of the present disclosure;
FIGs. 10B-10E are schematic diagrams illustrating an exemplary process of performing a precise segmentation after sliding window according to some embodiments of the present disclosure;
FIG. 11 is a comparison diagram illustrating exemplary effects of segmentation results according to some embodiments of the present disclosure;
FIG. 12 is a flowchart illustrating an exemplary process of registering a first segmentation image with a second segmentation image according to some embodiments of the present disclosure;
FIGs. 13-14 are flowcharts illustrating an exemplary process of determining a registration deformation field according to some embodiments of the present disclosure;
FIG. 15 is a schematic diagram illustrating an exemplary process of obtaining a first segmentation image and a second segmentation image by segmentation according to some embodiments of the present disclosure;

FIG. 16 is a flowchart illustrating an exemplary method for an image-assisted interventional surgery according to some embodiments of the present disclosure;

FIG. 17A is a flowchart illustrating an exemplary mask fusion algorithm according to some embodiments of the present disclosure;

FIG. 17B is a flowchart illustrating an exemplary process of determining a first intersection and adjusting an element mask based on the first intersection according to some embodiments of the present disclosure;

FIG. 18 is a flowchart illustrating an exemplary process of performing a connected domain processing on a fat-free mask according to some embodiments of the present disclosure;

FIG. 19 is a flowchart illustrating an exemplary process of obtaining a processed fat-free mask based on a fat-free mask after the connected domain processing according to some embodiments of the present disclosure;

FIG. 20 is a comparison diagram illustrating an exemplary effect of fusing a fat-free mask with a target organ mask according to some embodiments of the present disclosure;

FIG. 21 is a flowchart illustrating an exemplary process of combining a precise segmentation with a fast segmentation according to some embodiments of the present disclosure;

FIG. 22 is a comparison diagram illustrating exemplary effects of fusing a precise segmentation result mask with a fast segmentation result mask according to some embodiments of the present disclosure; and

FIG. 23 is a block diagram illustrating an exemplary a system for an image-assisted interventional surgery according to some embodiments of the present disclosure.

## DETAILED DESCRIPTION

In order to more clearly illustrate the technical solutions of the embodiments of the present disclosure, the accompanying drawings required to be used in the description of the embodiments are briefly described below. Obviously, the accompanying drawings in the following description are only some examples or embodiments of the present disclosure, and it is possible for a person of ordinary skill in the art to apply the present disclosure to other similar scenarios in accordance with these drawings without creative labor. Unless obviously obtained from the context or the context illustrates otherwise, the same numeral in the drawings refers to the same structure or operation.

It should be understood that the terms "system," "device," "unit" and/or "module" used herein are a way to distinguish between different components, elements, parts, sections, or assemblies at different levels. However, the terms may be replaced by other expressions if other words accomplish the same purpose.

As shown in the present disclosure and in the claims, unless the context clearly suggests an exception, the words "one," "a," "an," "one kind," and/or "the" do not refer specifically to the singular, but may also include the plural. Generally, the terms "including" and "comprising" suggest only the inclusion of clearly identified steps and elements, however, the steps and elements that do not constitute an exclusive list, and the method or apparatus may also include other steps or elements.

Flowcharts are used in the present disclosure to illustrate the operations performed by a system according to embodiments of the present disclosure, and the related descriptions are provided to aid in a better understanding of the magnetic resonance imaging method and/or system. It should be appreciated that the preceding or following operations are not necessarily performed in an exact sequence. Instead, steps can be processed in reverse order or simultaneously. Also, it is possible to add other operations to these processes or to remove a step or steps from these processes.

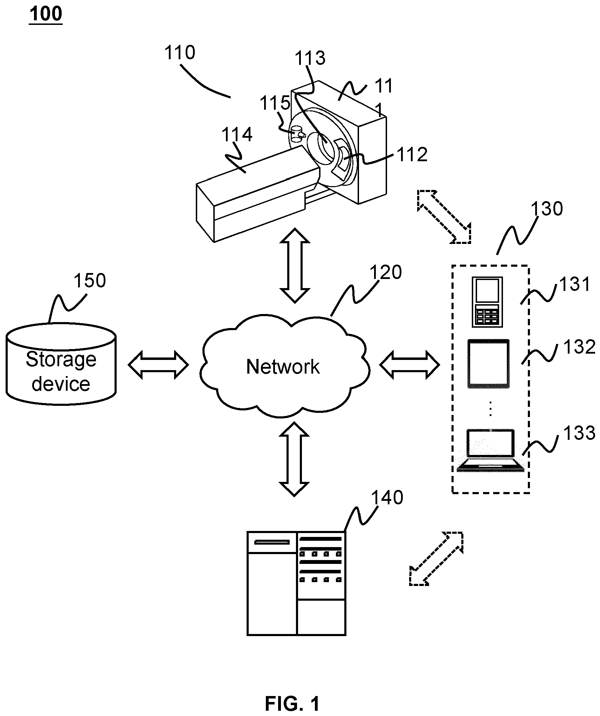

FIG. 1 is a schematic diagram illustrating an application scenario of a system for an image-assisted interventional surgery according to some embodiments of the present disclosure.

In some embodiments, a system 100 for an image-assisted interventional surgery may be applied to various interventional surgeries/interventional treatments. In some embodiments, the various interventional surgeries/interventional treatments may include a cardiovascular interventional surgery, a tumor interventional surgery, an obstetrical and gynecological interventional surgery, a musculoskeletal interventional surgery, or any other feasible interventional surgery, such as a neurological interventional surgery. In some embodiments, the various interventional surgeries/interventional treatments may include percutaneous puncture biopsy, coronary angiography, thrombolytic therapy, stent implantation, or any other feasible interventional surgery, such as an ablation surgery.

The system 100 for the image-assisted interventional surgery may include a medical scanning device 110, a network 120, one or more terminals 130, a processing device 140, and a storage device 150. Connections between components of the system 100 for the image-assisted interventional surgery may be variable. As shown in FIG. 1, the medical scanning device 110 may be connected with the processing device 140 via the network 120. As another example, the medical scanning device 110 may be directly connected with the processing device 140, as indicated by dotted double-sided arrows connecting the medical scanning device 110 and the processing device 140. As another example, the storage device 150 may be connected with the processing device 140 directly or via the network 120. Merely by way of example, the one or more terminals 130 may be directly connected with the processing device 140 (as indicated by dotted arrows connecting the one or more terminals 130 and the processing device 140) or may be connected with the processing device 140 via the network 120.

The medical scanning device 110 may be configured to scan a scan subject using high-energy rays (e.g., X-rays, gamma rays, etc.) to collect scan data related to the scan subject. The scan data may be configured to generate one or more images of the scan subject. In some embodiments, the medical scanning device 110 may include an ultrasound imaging (US) device, a computed tomography (CT) scanner, a digital radiography (DR) scanner (e.g., a mobile digital radiography scanner), a digital subtraction angiography (DSA) scanner, a dynamic spatial reconstruction (DSR) scanner, an X-ray microscope scanner, a multimodal scanner, or the like, or any combination thereof. In some embodiments, the multimodal scanner may include a computed tomography-positron emission tomography (CT-PET) scanner and a computed tomography-magnetic resonance imaging (CT-MRI) scanner. The scan subject may be biological or non-biological. Merely by way of example, the scan subject may include a patient, an artificial object (e.g., an artificial phantom), etc. As another example, the scan subject may include a specific part, an organ, and/or tissues of the patient.

As shown in FIG. 1, the medical scanning device 110 may include a gantry 111, a detector 112, a detection region 113, a table 114, and a radioactive source 115. The gantry 111 may be configured to support the detector 112 and the radioactive source 115. The scan subject may be placed on the table 114 for scanning. The radioactive source 115 may be configured to emit radioactive rays to the scan subject. The detector 112 may be configured to detect the radioactive rays (e.g., X-rays) emitted from the radioactive source 115. In some embodiments, the detector 112 may include one or more detector units. The one or more detector units may include a scintillation detector (e.g., a cesium iodide detector), a gas detector, etc. The one or more detector units may include a single-row detector and/or a multi-row detector.

The network 120 may include any suitable network that facilitates the exchange of information and/or data of the system 100 for the image-assisted interventional surgery. In some embodiments, one or more components (e.g., the medical scanning device 110, the one or more terminals 130, the processing device 140, and the storage device 150) of the system 100 for the image-assisted interventional surgery may exchange information and/or data with each other via the network 120. For example, the processing device 140 may obtain image data from the medical scanning device 110 via the network 120. As another example, the processing device 140 may obtain user instructions from the terminal 130 via the network 120.

The network 120 may be or may include a public network (e.g., the Internet), a private network (e.g., a local area network (LAN), a wide area network (WAN), etc.), a wired network (e.g., an Ethernet network, a wireless network (e.g., an 802.11 network, a Wi-Fi network, etc.), a cellular network (e.g., a Long Term Evolution (LTE) network), a frame relay network, a virtual private network ("VPN"), a satellite network, a telephone network, a router, a hub, a switch, a server computer, or the like, or any combination thereof. Merely by way of example, the network 120 may include a cable network, a wired network, a fiber optic network, a telecommunication network, an intranet, a wireless local area network (WLAN), a metropolitan area network (MAN), a public switched telephone network (PSTN), a Bluetooth™ network, a ZigBee™ network, a near field communication (NFC) network, or the like, or any combination thereof. In some embodiments, the network 120 may include one or more network access points. For example, the network 120 may include wired and/or wireless network access points such as a base station and/or an Internet exchange point via which the one or more components of the system 100 for the image-assisted interventional surgery may be connected to the network 120 to exchange data and/or information.

The one or more terminals 130 may include a mobile device 131, a tablet computer 132, a laptop computer 133, or the like, or any combination thereof. In some embodiments, the mobile device 131 may include a smart home device, a wearable device, a mobile device, a virtual reality (VR) device, an augmented reality (AR) device, or the like, or any combination thereof. In some embodiments, the smart home device may include a smart lighting device, a control device for a smart electrical device, a smart monitoring device, a smart TV, a smart camera, an intercom, or the like, or any combination thereof. In some embodiments, the mobile device 131 may include a mobile phone, a personal digital assistant (PDA), a gaming device, a navigation device, a point of sale (POS) device, a laptop computer, a tablet computer, a desktop computer, or the like, or any combination thereof. In some embodiments, the VR device and/or the AR device may include a VR helmet, VR glasses, VR goggles, an AR helmet, AR glasses, AR goggles, or the like, or any combination thereof. For example, the VR device and/or the AR device may include Google Glass™, Oculus Rift™, Hololens™, Gear VR™, or the like. In some embodiments, the one or more terminals 130 may be a portion of the processing device 140.

The processing device 140 may process the data and/or information obtained from the medical scanning device 110, the one or more terminals 130, and/or the storage device 150. For example, the processing device 140 may obtain data obtained by the medical scanning device 110, generate a medical image (e.g., a pre-operative image with contrast and an intra-operative image without contrast) using the data, and generate segmentation result data (e.g., a first segmentation image, a second segmentation image, spatial positions of blood vessels and lesions during the surgery, a registration map, a fat-free mask, etc.) by segmenting the medical image. As another example, the processing device 140 may obtain the medical image, planning mode data (e.g., fast segmentation mode data and/or precise segmentation mode data), and/or a scanning protocol from the one or more terminals 130.

In some embodiments, the processing device 140 may be a single server or a server group. The server group may be centralized or distributed. In some embodiments, the processing device 140 may be local or remote. For example, the processing device 140 may access information and/or data stored in the medical scanning device 110, the one or more terminals 130, and/or the storage device 150 via the network 120. As another example, the processing device 140 may be directly connected with the medical scanning device 110, the one or more terminals 130, and/or the storage device 150 to access the stored information and/or data. In some embodiments, the processing device 140 may be implemented on a cloud platform.

The storage device 150 may be configured to store data, instructions, and/or any other information. In some embodiments, the storage device 150 may store data obtained from the medical scanning device 110, the one or more terminals 130, and/or the processing device 140. For example, the storage device 150 may store medical image data (e.g., the pre-operative image with contrast, the intra-operative image without contrast, the first segmentation image, the second segmentation image, etc.), and/or positioning information data obtained from the medical scanning device 110. As another example, the storage device 150 may store the medical image and/or the scanning protocol input from the one or more terminals 130. As a further example, the storage device 150 may store data (e.g., the medical image data, organ mask data, the positioning information data, result data after precise segmentation, data related to the spatial positions of the blood vessels and the lesions during the surgery, the registration map, the fat-free mask, etc.) generated by the processing device 140.

In some embodiments, the storage device 150 may store data and/or instructions that the processing device 140 executes or uses to execute the exemplary methods described in the present disclosure. In some embodiments, the storage device 150 may include a mass storage device, a removable storage device, a volatile read-write memory, a read-only memory (ROM), or the like, or any combination thereof. Exemplary mass storage devices may include a magnetic disk, an optical disk, a solid-state drive, or the like. Exemplary removable storage devices may include a flash drive, a floppy disk, an optical disk, a memory card, a compressed disk, a tape, or the like. Exemplary volatile read-write memories may include a random access memory (RAM). Exemplary RAMs may include a dynamic random access memory (DRAM), a double data rate synchronous dynamic access memory (DDR SDRAM), a static random access memory (SRAM), a thyristor random access memory (T-RAM), a zero capacitance random access memory (Z-RAM), or the like. Exemplary ROMs may include a mask read-only memory (MROM), a programmable read-only memory (PROM), an erasable programmable read-only memory (EPROM), an electrically erasable programmable read-only memory (EEPROM), a compact disk read-only memory (CD-ROM), a digital versatile disk redistribution memory, or the like. In some embodiments, the storage device 150 may be implemented on the cloud platform.

In some embodiments, the storage device 150 may be connected to the network 120 to communicate with one or more other components (e.g., the processing device 140, and the one or more terminals 130) of the system 100 for the image-assisted interventional surgery. The one or more components of the system 100 for the image-assisted interventional surgery may access the data or the instructions stored in the storage device 150 via the network 120. In some embodiments, the storage device 150 may be directly connected with or communicate with one or more other components (e.g., the processing device 140, and the one or more terminals 130) of the system 100 for the image-assisted interventional surgery. In some embodiments, the storage device 150 may be a portion of the processing device 140.

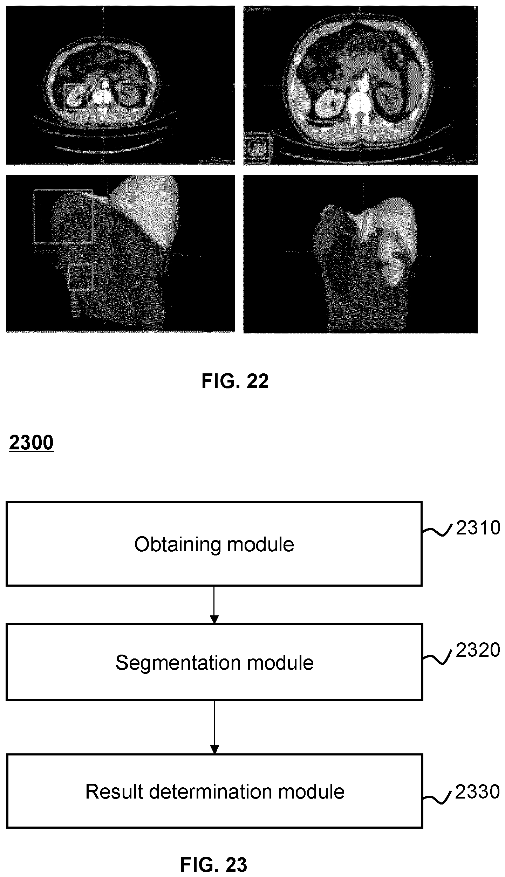

The description of the system 100 for the image-assisted interventional surgery is intended to be illustrative, not to limit the scope of the present disclosure. Many substitutions, modifications, and variations are apparent to those having ordinary skills in the art. It is understood that, for those having ordinary skills in the art, after understanding the principle of the system, it is possible to arbitrarily combine the various modules or to form a subsystem connected to other modules without departing from this principle. In some embodiments, an obtaining module 2310, a segmentation module 2320, and a result determination module 2330 of FIG. 23 may be different modules in a system, or a module may implement the functions of two or more of the above modules. For example, each module may share a storage module, or each module may have its own storage module. The features, structures, methods, and other features of the exemplary embodiments described in the present disclosure may be combined in various ways to obtain additional and/or alternative exemplary embodiments. For example, the processing device 140 and the medical scanning device 110 may be integrated into a single device. Such variations are within the scope of protection of the present disclosure.

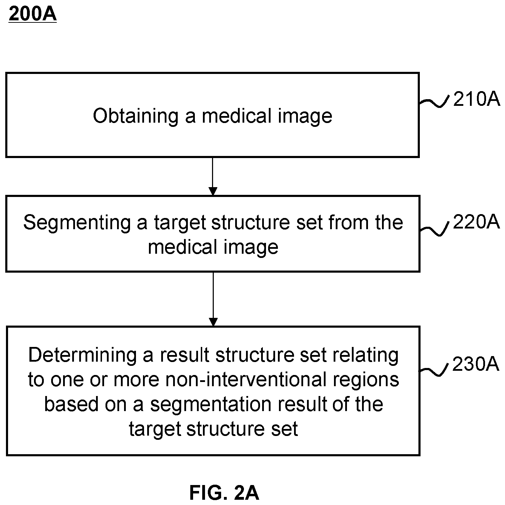

FIG. 2A is a flowchart illustrating an exemplary method for an image-assisted interventional surgery according to some embodiments of the present disclosure. As shown in FIG. 2A, a process 200A may include the following operations.

In 210A, a medical image may be obtained. In some embodiments, the operation 210A may be performed by the obtaining module 2310 or the medical scanning device 110.

The medical image refers to a medical image generated based on various imaging mechanisms. In some embodiments, the medical image may be a three-dimensional (3D) medical image. In some embodiments, the medical image may be a two-dimensional (2D) medical image. In some embodiments, the medical image may include a CT image, a PET -CT image, a US image, or an MR image.

In some embodiments, a medical image of a scan subject may be obtained. In some embodiments, the medical image of the scan subject, such as the CT image, may be obtained from the medical scanning device 110. In some embodiments, the medical image of the scan subject, such as the MR image, may be obtained from the one or more terminals 130, the processing device 140, and the storage device 150.

In some embodiments, the scan subject may include a biological scan subject or a non-biological scan subject. In some embodiments, the biological scan subject may include a patient, or a specific part, an organ, and/or tissues of the patient, such as the abdomen, the chest, or a tumor tissue, etc. In some embodiments, the non-biological scan subject may include an artificial object, such as an artificial phantom, etc.

It should be noted that in some embodiments, the medical image may also be obtained in any other feasible way. For example, the medical image may be obtained from a cloud server and/or a medical system (e.g., a medical system center of a hospital, etc.) via the network 120, which is not limited in the embodiments of the present disclosure.

In some embodiments, the medical image may include a pre-operative image with contrast, which refers to an image obtained by scanning a scan subject (e.g., the patient, etc.) through a medical scanning device after a contrast agent is injected before a surgery. In some embodiments, the medical image may include an intra-operative image without contrast. The intra-operative image without contrast refers to an image obtained by performing plain scanning on the scan subject through the medical scanning device during the surgery. In some embodiments, the intra-operative image without contrast may be a real-time scan image. In some embodiments, the intra-operative image without contrast includes an image that is acquired during a surgical preparation process before the surgery is performed (i.e., before a needle is actually inserted).

In 220A, a target structure set may be segmented from the medical image. In some embodiments, the operation 220A may be performed by the segmentation module 2320.

The target structure set refers to a part, an organ, and/or a tissue to be segmented in the medical image. In some embodiments, the target structure set may include a plurality of elements, such as one or more of a target organ, a blood vessel in the target organ, fat, a thoracic cavity/an abdominal cavity, etc. In some embodiments, the target organ may include a lung, a liver, a spleen, a kidney, or any other possible organ tissue, such as a thyroid gland, etc.

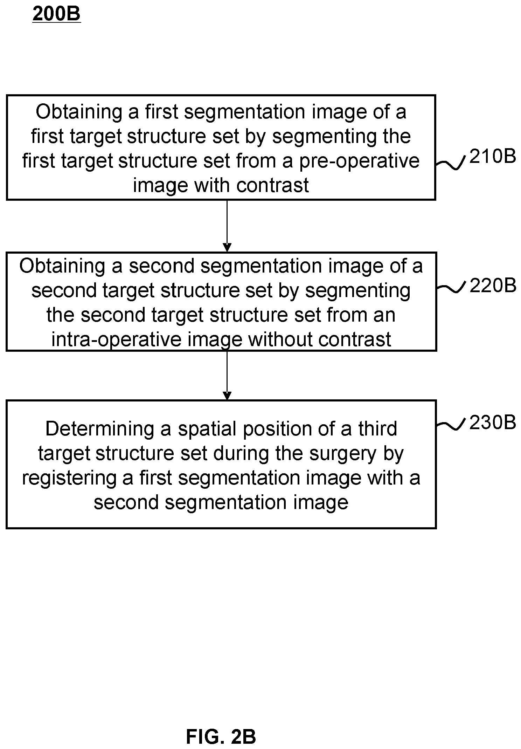

In some embodiments, the target structure set may include a first target structure set of the pre-operative image with contrast. The first target structure set of the pre-operative image with contrast may include blood vessels in the target organ to be treated. In some embodiments, the first target structure set of the pre-operative image with contrast may include the target organ and a lesion in addition to the blood vessels in the target organ. In some embodiments, a segmentation result (e.g., a first segmentation image) may be obtained by segmenting the first target structure set from the pre-operative image with contrast. More descriptions description regarding a process of segmenting the first target structure set from the pre-operative image with contrast may be found in FIGs. 2B-15 and related descriptions thereof.

In some embodiments, the target structure set may include a second target structure set of the intra-operative image without contrast. One or more regions or organs included in the second target structure set of the intra-operative image without contrast may be determined based on a planning mode (e.g., a fast segmentation mode and/or a precise segmentation mode) of an interventional surgery. That is, for different planning modes of the interventional surgery, the one or more regions or organs included in the second target structure set may be different. For example, when the planning mode is the fast segmentation mode, the second target structure set may include one or more non-interventional regions. As another example, when the planning mode is the precise segmentation mode, the second target structure set may include all important organs in the intra-operative image without contrast. The important organs refer to organs that need to be avoided in an interventional planning path during the interventional surgery, such as the liver, the kidneys, and blood vessels outside the target organ. In some embodiments, the second target structure set may include the target organ and the lesion in addition to the one or more non-interventional regions/all the important organs in the intra-operative image without contrast. In some embodiments, a segmentation result (e.g., a second segmentation image) may be obtained by segmenting the second target structure set from the intra-operative image without contrast. More descriptions description regarding the segmentation of the second target structure set from the intra-operative image without contrast may be found in FIGs. 2B-15 and related descriptions thereof.

In some embodiments, after the medical image is obtained in the operation 210A, the medical image may be preprocessed, and each element of the target structure set may be segmented from the preprocessed image. In some embodiments, the preprocessing may include an image preprocessing operation. In some embodiments, the image preprocessing operation may include a normalization processing and/or a background removal processing.

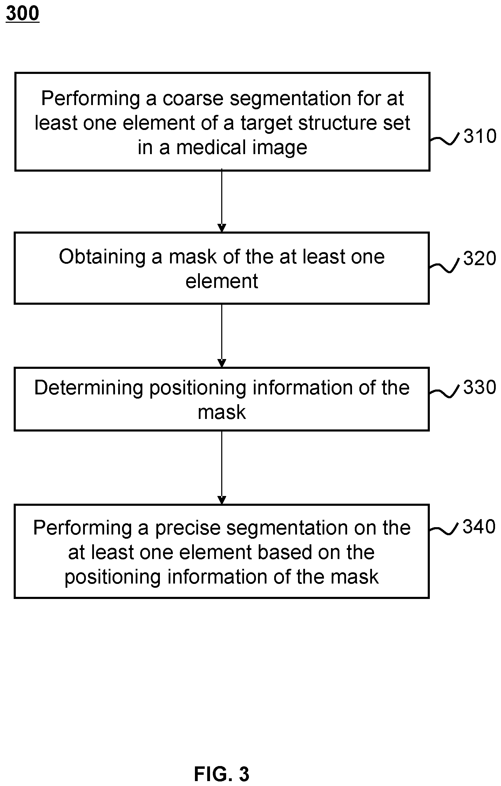

In some embodiments, an image segmentation method may include a threshold segmentation manner, a regional growth manner, or a level set manner. In some embodiments, the target structure set may be segmented from the medical image using a manner based on a deep learning convolutional network. In some embodiments, the manner based on the deep learning convolutional network may include a segmentation manner based on a fully convolutional network, such as U-net, etc. More descriptions regarding the segmentation manner may be found in FIGs. 3-11 and related descriptions thereof.

In 230A, a result structure set relating to the one or more non-interventional regions may be determined based on the segmentation result of the target structure set. In some embodiments, the operation 230A may be performed by the result determination module 2330.

The one or more non-interventional regions refer to regions that need to be avoided in the interventional planning path during the interventional surgery. In some embodiments, the one or more non-interventional regions may include a non-interventional region, a non-introduction or non-implantation region, and a non-injection region. In some embodiments, the one or more non-interventional regions may include an organ, a blood vessel, a bone, etc.

The segmentation result of the target structure set may be obtained after the segmentation of the target structure set is performed on the medical image. In some embodiments, the segmentation result of the target structure set may include an element mask. By segmenting the target structure set from the medical image, an element mask corresponding to each element of the target structure set may be obtained. In some embodiments, by segmenting the target structure set from the medical image, one or more of a target organ mask, a blood vessel mask in the target organ, a fat mask, a thoracic cavity/abdominal cavity mask, etc., may be obtained. In some embodiments, the thoracic cavity/the abdominal cavity is collectively referred to as a thoracic-abdominal region, and the corresponding thoracic cavity/abdominal cavity mask is referred to as a thoracic-abdominal mask. The element mask may be a pixel-level classification label. Taking an abdominal medical image as an example, the element mask represents categorization of pixels in the medical image. For example, the pixels in the medical image may be categorized as a background, the liver, the spleen, the kidneys, etc. A summary region corresponding to a specific category may be represented by a specific label value. For example, all pixels categorized as the liver may be summarized, and the summary region may be represented by a label value corresponding to the liver. The label value may be set according to a specific segmentation task.

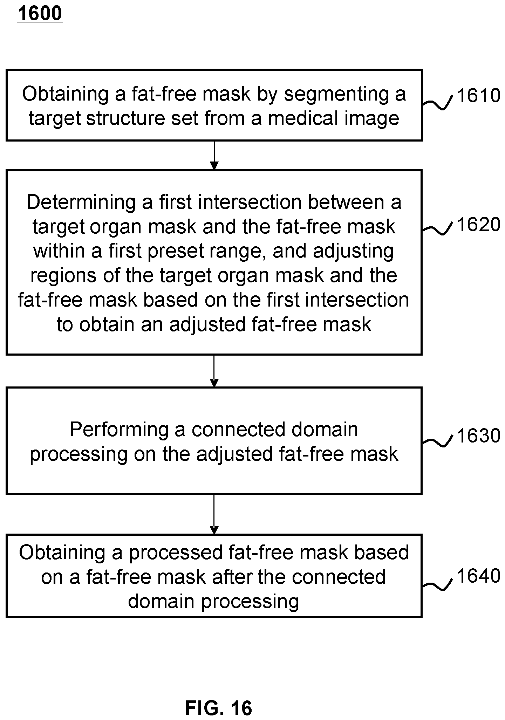

In some embodiments, the result structure set may include a fat-free mask. In some embodiments, the fat mask and the thoracic-abdominal mask may be obtained by segmenting the target structure set from the medical image. In some embodiments, the fat-free mask may be obtained based on the fat mask and the thoracic-abdominal mask. In some embodiments, the fat mask may be subtracted from the thoracic-abdominal mask to obtain the fat-free mask for the chest and the abdomen. In interventional surgery, a fat region in a thoracic cavity/abdominal cavity region may be regarded as an interventional region. The fat-free mask region may be a non-interventional region. More descriptions regarding the fat-free mask may be found elsewhere in the present disclosure (e.g., FIGs. 16-22 and related descriptions thereof).

In some embodiments, the segmentation result of the target structure set may include a segmentation image. In some embodiments, a first segmentation image of the first target structure set may be obtained by segmenting the first target structure set from the pre-operative image with contrast. The first segmentation image refers to a segmentation image of the first target structure set (e.g., the target organ, and the blood vessels and the lesion in the target organ in the pre-operative image with contrast) obtained by segmenting the pre-operative image with contrast. In some embodiments, a second segmentation image of the second target structure set may be obtained by segmenting the second target structure set from the intra-operative image without contrast. The second segmentation image refers to a segmentation image of the second target structure set (e.g., the one or more non-interventional regions or important organs, the target organ, and the lesion) obtained by segmenting the intra-operative image without contrast.

In some embodiments, the result structure set may include a third target structure set during the surgery. In some embodiments, a spatial position of the third target structure set during the surgery may be determined based on the first segmentation image and the second segmentation image. For example, the spatial position of the third target structure set during the surgery may be determined by registering the first segmentation image with the second segmentation image. The third target structure set refers to a structural set obtained after the first segmentation image is registered with the second segmentation image. The third target structure set can more comprehensively and accurately reflect a current condition of the scan subject (e.g., the patient). The path of the interventional surgery may be planned based on the spatial position of the third target structure set such that a puncture needle can effectively avoid one or more non-interventional regions and/or all important organs and successfully reach the lesion. More descriptions regarding the segmentation image and the third target structure set may be found elsewhere in the present disclosure (e.g., FIGs. 2B-15 and related descriptions thereof).

It should be noted that, as described above, the result structure set (e.g., the fat-free mask) may be the non-interventional region. In some embodiments, the result structure set may be an interventional region. In this case, the result structure set needs to be subtracted from the total region to determine the non-intervention region.

It should be noted that the above description of the process 200A is only for example and explanation, and does not limit the scope of application of the present disclosure. For those skilled in the art, various modifications and changes can be made to the process 200A under the guidance of the present disclosure. However, these modifications and changes are still within the scope of protection of the present disclosure.

FIG. 2B is a flowchart illustrating an exemplary method for an image-assisted interventional surgery according to some embodiments of the present disclosure. As shown in FIG. 2B, a process 200B may include the following operations.

In 210B, a first segmentation image of a first target structure set may be obtained by segmenting the first target structure set from a pre-operative image with contrast. In some embodiments, the operation 210B may be performed by the segmentation module 2320.

In some embodiments, the first target structure set of the pre-operative image with contrast may include blood vessels in a target organ. In some embodiments, the first target structure set of the pre-operative image with contrast may include the target organ and a lesion in addition to the blood vessels in the target organ. In some embodiments, the target organ may include the brain, lungs, the liver, the spleen, kidneys, or any other possible organ tissues, such as the thyroid gland. The first segmentation image refers to a segmentation image of the first target structure set (e.g., the target organ, the blood vessels and the lesion in the target organ in the pre-operative image with contrast) obtained by segmenting the pre-operative image with contrast.

In 220B, a second segmentation image of a second target structure set may be obtained by segmenting the second target structure set from an intra-operative image without contrast. In some embodiments, the operation 220B may be performed by the segmentation module 2320.

In some embodiments, one or more regions or organs included in the second target structure set of the intra-operative image without contrast may be determined based on a planning mode (e.g., a fast segmentation mode and/or a precise segmentation mode) of the interventional surgery. That is, for different planning modes of the interventional surgery, the one or more regions or organs included in the second target structure set may be different. For example, when the planning mode is the fast segmentation mode, the second target structure set may include one or more non-interventional regions. As another example, when the planning mode is the precise segmentation mode, the second target structure set may include all important organs in the intra-operative image without contrast. The important organs refer to organs that need to be avoided in an interventional planning path during the interventional surgery, such as the liver, the kidneys, and blood vessels outside the target organ. In some embodiments, the second target structure set may also include the target organ and the lesion in addition to the one or more non-interventional regions/all the important organs in the intra-operative image without contrast. The second segmentation image refers to a segmentation image of the second target structure set (e.g., the one or more non-interventional regions or important organs, the target organ, and the lesion) obtained by segmenting the intra-operative image without contrast.

In some embodiments, the first target structure set and the second target structure set may have an intersection. For example, when the first target structure set includes the blood vessels in the target organ and the target organ, and the second target structure set includes the one or more non-intervention regions (or all the important organs), the target organ, and the lesion, the intersection between the first target structure set and the second target structure set may be the target organ. As another example, when the first target structure set includes the blood vessels in the target organ, the target organ, and the lesion, and the second target structure set includes the one or more non-intervention region (or all the important organs), the target organ, and the lesion, the intersection between the first target structure set and the second target structure set may be the target organ and the lesion.

In some embodiments, the planning mode of the interventional surgery may be obtained before the operation 220B is performed.

The interventional surgery (or an interventional therapy) is a minimally invasive treatment surgery performed using modern high-tech means. Specifically, interventional surgery is a medical means of diagnosing and locally treating a pathological condition in a body by introducing a special catheter, a guidewire, and other precision instruments into the human body under the guidance of a medical scanning device or a medical imaging device. In some embodiments, the interventional surgery may be an interventional surgery in an actual diagnosis and treatment stage (for a patient), or an interventional surgery in an animal experiment or a simulation stage, which is not limited in the embodiments of the present disclosure.

In some embodiments, the planning mode may be a planning mode for segmenting a scan image during the surgery. In some embodiments, the planning mode may include the fast segmentation mode and/or the precise segmentation mode.

In some embodiments, in the operation 220B, segmenting the second target structure set of the intra-operative image without contrast may include: segmenting a fourth target structure set of the intra-operative image without contrast according to the planning mode. In some embodiments, the fourth target structure set of the intra-operative image without contrast may be segmented according to at least one of the fast segmentation mode or the precise segmentation mode.

In some embodiments, the fourth target structure set may be a portion of the second target structure set, such as the one or more non-interventional regions, and all the important organs outside the target organ. In different planning modes, the fourth target structure set may include different regions/organs. In some embodiments, in the fast segmentation mode, the fourth target structure set may include the one or more non-interventional regions. In some embodiments, in the precise segmentation mode, the fourth target structure set may include preset important organs.

In some embodiments, in the fast segmentation mode, region positioning computation may be performed on the intra-operative image without contrast, and segmentation and extraction may be performed on the one or more non-interventional regions.

In some embodiments, a post-processing may be performed on regions outside the one or more non-interventional regions and the target organ to ensure that there is no hollow region between the one or more non-interventional regions and the target organ. The hollow region refers to a background region enclosed by boundaries connected by foreground pixels. In some embodiments, the one or more non-interventional regions may be obtained by subtracting the target organ and the interventional region from the abdominal cavity (or the thoracic cavity) region. For example, a fat mask may be subtracted from a thoracic-abdominal mask to obtain a fat-free mask. In this case, since the target organ is located in a thoracic-abdominal region, the fat-free mask may include a target organ mask. As another example, the target organ mask may be subtracted from the thoracic-abdominal mask to obtain a thoracic-abdominal target removal mask, and then the fat mask may be subtracted from the thoracic-abdominal target removal mask to obtain the fat-free mask. At this time, the fat-free mask may not include the target organ mask. After the one or more non-interventional regions are obtained by subtracting the target organ and the interventional region from the abdominal cavity (or the thoracic cavity) region, the hollow region may exist between the target organ and the one or more non-interventional regions, the hollow region belonging to neither the target organ nor the one or more non-interventional regions. In this case, the post-processing may be performed on the hollow region to complete the hollow region. The hollow region after the post-processing operation may be regarded as a non-interventional region. In some embodiments, the post-processing may include an erosion operation and an expansion operation. In some embodiments, the erosion operation and the expansion operation may be implemented by performing convolution operation on the intra-operative image without contrast using a filter. In some embodiments, the erosion operation may be performed after the filter is convolved with the intra-operative image without contrast, a local minimum value is obtained according to a preset erosion range, such that a contour of the intra-operative image without contrast is reduced to a desired range, and a target highlight region in an initial image displayed in the intra-operative image without contrast is reduced by a certain range. In some embodiments, the expansion operation may be performed after the filter is convolved with the intra-operative image without contrast, a local maximum value is obtained according to the preset erosion range, such that the contour of the intra-operative image without contrast is expanded to a desired range, and the target highlight region in the initial image displayed in the intra-operative image without contrast is reduced by a certain range.

In some embodiments, in the fast segmentation mode, the region positioning may be performed on the intra-operative image without contrast, and then the segmentation and extraction may be performed on the one or more non-interventional regions. In some embodiments, a blood vessel mask inside the target organ may be determined based on a segmentation mask and a blood vessel mask of the target organ in the intra-operative image without contrast. It should be noted that in the fast segmentation mode, only the blood vessels inside the target organ need to be segmented; in the precise segmentation mode, the blood vessels inside the target organ and other external blood vessels both need be segmented.

A mask (e.g., an organ mask) may be a pixel-level categorization label. Taking an abdominal medical image as an example, the mask represents the categorization of pixels in the medical image. For example, the pixels in the medical image may be categorized as the background, the liver, the spleen, kidneys, etc. A summary region of a specific category may be represented by a specific label value. For example, all pixels categorized as the liver may be summarized, and the summary region may be represented by a label value corresponding to the liver. The label value here may be set according to a specific coarse segmentation task. The segmentation mask refers to a corresponding mask obtained after a segmentation operation. In some embodiments, the mask may include an organ mask (e.g., an organ mask of the target organ) and a blood vessel mask.

In some embodiments, in the fast segmentation mode, the thoracic region or the abdominal region is taken as an example, the region positioning may be performed on the thoracic region or the abdominal region within a scanning range of the intra-operative image without contrast. For example, for the abdominal cavity, a region from the top of the liver to the bottom of the rectum may be taken as a positioning region of the abdominal cavity; and for the thoracic cavity, a region from the top of the esophagus to the bottom of the lungs (or the top of the liver) may be taken as a positioning region of the thoracic cavity. After region positioning information of the thoracic cavity or abdominal cavity region is determined, the abdominal cavity or the thoracic cavity may be segmented, and the segmented region may be re-segmented to extract the interventional region (which is opposed to the one or more non-interventional regions and includes a puncture region, a fat region, etc.). Finally, the segmentation mask of the target organ and the segmentation mask of the interventional region may be removed from a segmentation mask of the abdominal cavity or the thoracic cavity, and a segmentation mask of the one or more non-interventional regions may be obtained. In some embodiments, the interventional region may include a fat portion, such as a gap containing fat between two organs. Taking the liver as an example, a portion of a region between the subcutaneous and the liver may be covered by fat. Due to a fast processing speed in the fast segmentation mode, the planning speed is faster and the time is shorter, which improves the image processing efficiency. More descriptions regarding the method and the processing for segmentation and extraction of the one or more non-interventional regions in the fast segmentation mode may be found elsewhere in the present disclosure (e.g., FIGs. 16-22 and related descriptions thereof).

In some embodiments, in the precise segmentation mode, all organs in the intra-operative image without contrast may be segmented. In some embodiments, all organs in the intra-operative image without contrast may include one or more basic organs and one or more important organs in the intra-operative image without contrast. In some embodiments, the one or more basic organs in the intra-operative image without contrast may include the target organ in the intra-operative image without contrast. In some embodiments, in the precise segmentation mode, one or more preset important organs in the intra-operative image without contrast be segmented. The one or more preset important organs may be determined according to the importance of each of the organs in the intra-operative image without contrast. For example, all important organs in the intra-operative image without contrast may be used as the preset important organs. In some embodiments, a ratio of a total volume of the one or more preset important organs in the precise segmentation mode to a total volume of the one or more non-interventional regions in the fast segmentation mode may be less than a preset efficiency factor m. A difference in the preset efficiency factors corresponding to different segmentation modes m characterizes a difference in segmentation efficiency and/or segmentation fineness between different segmentation modes. By reasonably setting the preset efficiency factor m, the segmentation efficiency and the segmentation fineness of the interventional surgery can be controlled. In some embodiments, the preset efficiency factor m may be equal to or less than 1. The larger the value of the preset efficiency factor m, the higher the segmentation efficiency (or the higher the segmentation fineness) of the segmentation based on different segmentation modes; the smaller the value of the preset efficiency factor m, the lower the segmentation efficiency (or the lower the segmentation fineness) of the segmentation based on different segmentation modes. In some embodiments, the preset efficiency factor m may be set based on a type of interventional surgery. The type of the interventional surgery may include a urological surgery, a thoracic and abdominal surgery, a cardiovascular surgery, a gynecological and obstetric interventional surgery, a musculoskeletal surgery, etc. Merely by way of example, the preset efficiency factor m in the urological surgery may be set smaller; and the preset efficiency factor m in the thoracic and abdominal surgery may be set larger.

In some embodiments, the value of the preset efficiency factor m may not only affect the segmentation efficiency and/or the segmentation fineness, but also affect the time used for the segmentation process. For example, the larger the preset efficiency factor m, the larger the total volume of the one or more preset important organs in the precise segmentation mode, and the longer the time is needed for the precise segmentation. Therefore, the preset efficiency factor may be determined by comprehensively considering the segmentation time. In other words, the preset efficiency factor may be comprehensively determined based on the type of interventional surgery, the requirements for the segmentation efficiency, the requirements for the segmentation fineness, and the requirements for the segmentation time. In some embodiments, the preset efficiency factor m may be reasonably set based on big data and/or historical data. For example, for a certain type of interventional surgery, the segmentation efficiency and the segmentation time corresponding to different efficiency factors m of the type of interventional surgery may be collected in the big data and/or the historical data to determine a range of the preset efficiency factor m that the segmentation efficiency and the segmentation time can meet the requirements of the type of interventional surgery. In some embodiments, the range of the preset efficiency factor m may also be optimized and updated according to the feedback of a doctor, such that the updated range of the preset efficiency factor m can better meet the requirements for the interventional surgery and the segmentation time.

In some embodiments, when the preset efficiency factor m of a certain interventional surgery (referred to as a target surgery) is determined, a plurality of surgeries (referred to as comparison surgeries) with similar conditions (e.g., a similar surgery type) as the target surgery may be searched and determined in the big data and/or the historical data, and then a similarity between the target surgery and each of the plurality of comparison surgeries may be determined (e.g., the similarity may be determined based on the segmentation fineness, the segmentation time, etc.), so as to select a comparison surgery with a high similarity to the target surgery, and then the preset efficiency factor m of the target surgery may be determined according to the preset efficiency factor m of the comparison surgery. Merely by way of example, in the thoracic and abdominal interventional surgery, the range of the preset efficiency factor m may be in a range of 0.5-0.65.

In some embodiments, the preset efficiency factor m may be determined based on the big data and/or the historical data using a machine learning model. In some embodiments, an input of the machine learning model may include parameters of the interventional surgery. The parameters of the interventional surgery may include one or more of the type of interventional surgery, the segmentation efficiency, the segmentation time, etc. An output of the machine learning model may include a preset efficiency factor m. The machine learning model may be obtained by training based on the big data and/or the historical data. For example, an initial machine learning model may be trained using the big data and/or the historical data as training samples to obtain the machine learning model. In some embodiments, the machine learning model may include one or more of a linear classification model (LR), a support vector machine model (SVM), a naive Bayes model (NB), a K-nearest neighbor model (KNN), a decision tree model (DT), an integrated model (RF/GDBT, etc.), etc.

In some embodiments, in the precise segmentation mode, segmentation masks of all the important organs in the intra-operative image without contrast may be obtained by the segmentation. In some embodiments, in the precise segmentation mode, the segmentation masks and blood vessel masks of all the important organs in the intra-operative image without contrast may be obtained by the segmentation. In some embodiments, in the precise segmentation mode, blood vessel masks inside all the important organs may be determined based on the segmentation masks and the blood vessel masks of all the important organs in the intra-operative image without contrast. It can be seen that in the precise segmentation mode, the segmentation image is more detailed, which makes the planning path more selective, thereby enhancing the robustness of image processing.

FIG. 3 is a flowchart illustrating an exemplary segmentation process in a method for an image-assisted interventional surgery according to some embodiments of the present disclosure.

In some embodiments, a segmentation process 300 involved in the method for the image-assisted interventional surgery may include the following operations.

In 310, a coarse segmentation may be performed for at least one element of a target structure set in a medical image.

In 320, a mask of the at least one element may be obtained.

In 330, positioning information of the mask may be determined.

In 340, a precise segmentation may be performed on the at least one element based on the positioning information of the mask.

In some embodiments, the medical image may include a pre-operative image with contrast and an intra-operative image without contrast. The target structure set may include one or more of a first target structure set, a second target structure set, and a fourth target structure set.

In some embodiments, in the operation 310, the coarse segmentation operation may be performed on the at least one element of the target structure set in the medical image using a threshold segmentation manner, a regional growth manner, or a level set manner. The at least one element may include a target organ, blood vessels in the target organ, a lesion, one or more non-interventional regions, all important organs, etc., in the medical image. In some embodiments, performing the coarse segmentation based on the threshold segmentation manner may include: setting a plurality of different pixel threshold ranges, categorizing pixels in the medical image according to pixel values in the medical image, and segmenting pixels with pixel values within the same pixel threshold range as the same region. In some embodiments, performing the coarse segmentation based on the regional growth manner may include: setting a similarity determination condition according to a demand based on known pixels or a preset region including the known pixels in the medical image, comparing the pixels with surrounding pixels or comparing the preset region with surrounding regions based on the preset similarity determination condition, and fusing pixels or regions with a high similarity until the above process cannot be repeated, and then stopping the fusing to complete the coarse segmentation process. In some embodiments, the preset similarity determination condition may be determined according to preset image features, such as a grayscale, a texture, etc. In some embodiments, performing the coarse segmentation based on the level set manner may include: setting a target contour of the medical image to a zero level set of a high-dimensional function, differentiating the function, obtaining the target contour by extracting the zero level set from an output, and then segmenting a pixel region within a contour range from the target contour.

In some embodiments, the coarse segmentation may be performed on the at least one element of the target structure set in the medical image using a manner based on a deep learning convolutional network. In some embodiments, the manner based on the deep learning convolutional network may include a segmentation manner based on a full convolutional network. In some embodiments, the convolutional network may adopt a network framework based on a U-shaped structure, such as U-net, etc. In some embodiments, the network framework of the convolutional network may include an encoder, a decoder, and a skip connection structure. The encoder and the decoder may include a convolutional layer or a convolutional layer combined with an attention module, the convolutional layer being configured to extract features, the attention module being configured to pay more attention to a key region. The skip connection structure may be configured to combine features of different dimensions extracted by the encoder to the decoder, and finally output the segmentation result via the decoder. In some embodiments, when performing the coarse segmentation based on the deep learning convolutional network, the encoder of the convolutional neural network extracts features of the medical image through convolution; then the decoder of the convolutional neural network restores the features into a pixel-level segmentation probability map (which represents a probability that each pixel in the image belongs to a specific category), and finally outputs the segmentation probability map as a segmentation mask, thereby completing the coarse segmentation.

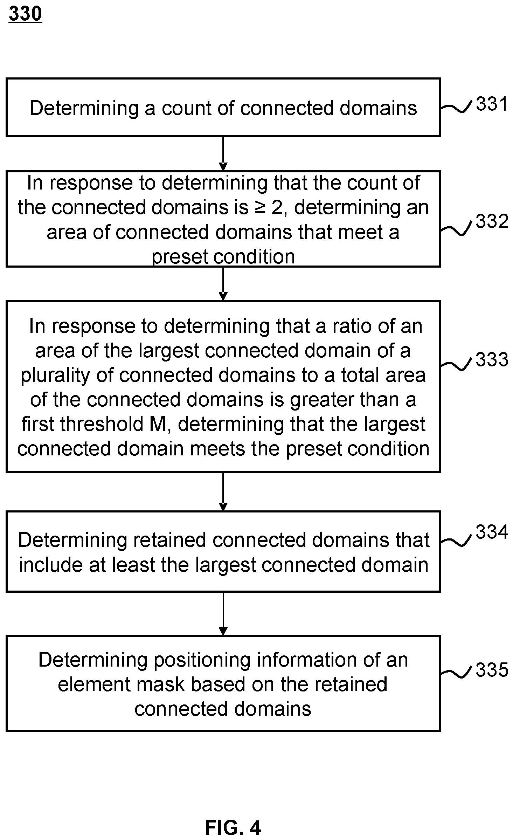

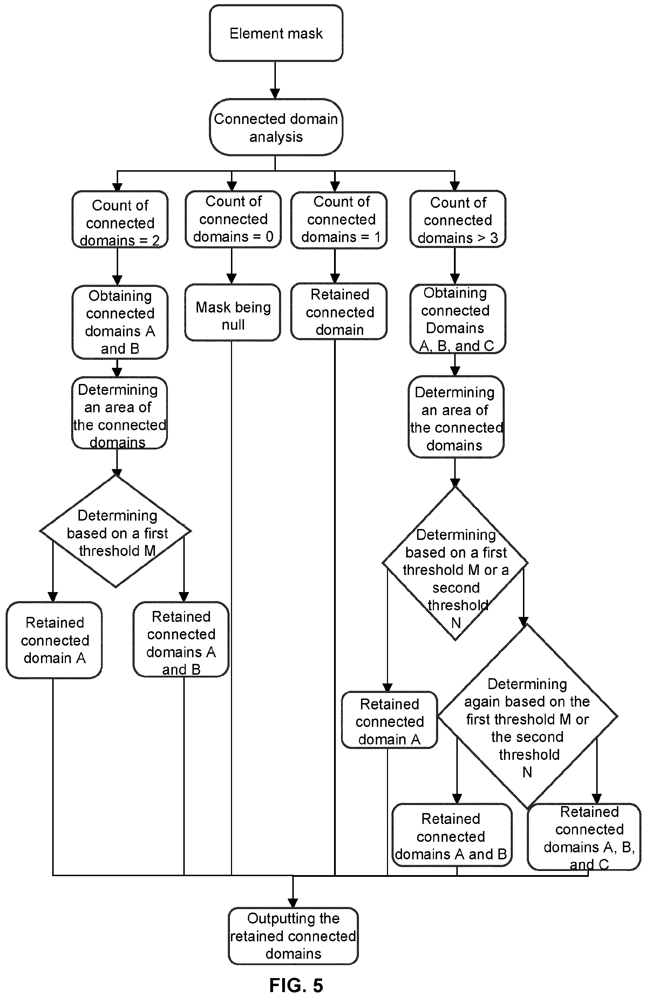

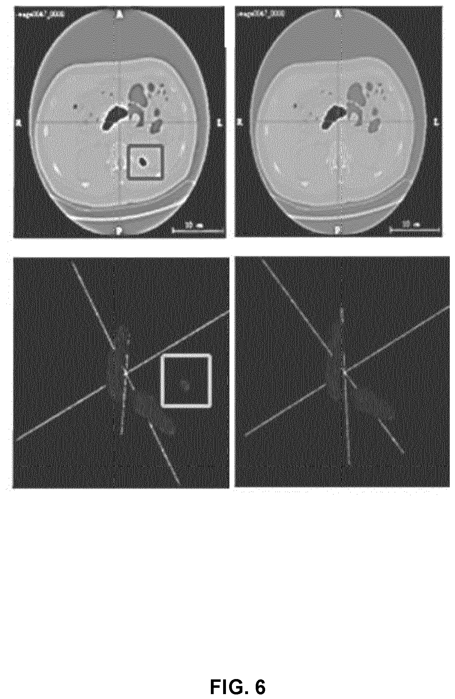

FIG. 4 is a flowchart illustrating an exemplary process of determining positioning information of an element mask according to some embodiments of the present disclosure. FIG. 5 is a flowchart illustrating an exemplary process of performing soft connected domain analysis on an element mask according to some embodiments of the present disclosure. FIG. 6 is a comparison diagram illustrating exemplary coarse segmentation effects of performing soft connected domain analysis on an element mask according to some embodiments of the present disclosure.

In some embodiments, in the operation 330, determining the positioning information of the element mask may include: performing soft connected domain analysis on the element mask. A connected domain, i.e., a connected region, generally refers to an image region including foreground pixel points with the same pixel value and adjacent positions in an image.

In some embodiments, in the operation 330, the performing the soft connected domain analysis on the element mask may include the following sub-operations.

In 331, a count of connected domains may be determined.

In 332, in response to determining that the count of the connected domains is greater than or equal to 2, an area of connected domains that meet a preset condition may be determined.

In 333, in response to determining that a ratio of an area of a largest connected domain in a plurality of connected domains to a total area of the plurality of connected domains is greater than a first threshold M, it is determined that the largest connected domain meets the preset condition.

In 334, retained connected domains that include at least the largest connected domain are determined.

In 335, the positioning information of the element mask may be determined based on the retained connected domains.

The preset condition refers to a condition that a connected domain needs to meet when the connected domain is used as a retained connected domain. For example, the preset condition may be a limiting condition on the area of the connected domain. In some embodiments, the medical image may include a plurality of connected domains, and the plurality of connected domains may have different areas. The plurality of connected domains with different areas may be sorted according to the size of the areas in a descending manner, and the sorted connected domains may be denoted as a first connected domain, a second connected domain, and a kth connected domain. The first connected domain may be the connected domain with the largest area of the plurality of connected domains, also referred to as the largest connected domain. In this case, the preset conditions corresponding to connected domains with different area ranks may be different. More descriptions may be found in the related descriptions of FIG. 5. In some embodiments, the connected domains that meet the preset condition may include: connected domains with top n areas in the sorting result. For example, when n is 3, whether each connected domain is a retained connected domain may be determined according to the order of the area rank and the corresponding preset condition. That is, whether the first connected domain is a retained connected domain may be determined first, and then whether the second connected domain is a retained connected domain may be determined. In some embodiments, n may be set based on a category of a target structure, such as a thoracic target structure and an abdominal target structure. In some embodiments, a value of the first threshold M may be in a range of 0.8-0.95. Within the value range, it can ensure that the soft connected domain analysis obtains an expected accuracy. In some embodiments, the value of the first threshold M may be in a range of 0.9-0.95, further improving the accuracy of the soft connected domain analysis. In some embodiments, the first threshold M may be set based on the category of the target structure, such as the thoracic target structure, and the abdominal target structure. In some embodiments, n/the first threshold M may also be reasonably set according to machine learning and/or big data, which is not limited here.

In some embodiments, in the operation 330, the performing the soft connected domain analysis on the element mask may include the following operations.

A count of the connected domains in the element mask and areas corresponding to the connected domains may be analyzed and determined based on the obtained element mask.

In some embodiments, in response to determining that the count of the connected domains is 0, it means that the corresponding mask is null, i.e., the mask obtaining or the coarse segmentation fails or a segmented subject does not exist, and no processing is performed. For example, for a patient who has received splenectomy, the mask of the spleen is null.

In some embodiments, in response to determining that the count of the connected domains is 1, it means that there is only one connected domain, no false positive, no segmentation or disconnection, etc., exist and the connected domain is retained. It can be understood that when the count of the connected domains is 0 and 1, there is no need to determine whether the connected domain is a retained connected domain according to the preset condition.

In some embodiments, in response to determining that the count of the connected domains is 2, connected domains A and B may be obtained according to the size of areas (S). An area of the connected domain A may be greater than an area of the connected domain B, i.e., S(A) > S(B). Referring to the above description, the connected domain A is also referred to as the first connected domain or the largest connected domain; and the connected domain B is referred to as the second connected domain. In some embodiments, in response to determining that the count of the connected domains is 2, the preset condition that the connected domains need to meet as the retained connected domains may relate to a relationship between a ratio of the area of the largest connected domain to a total area of the connected domains and a threshold. In a process of determining the connected domains, when a proportion of the area of the connected domain A to the total area of the connected domains A and B is greater than the first threshold M, i.e., S (A)/S(A+B) > the first threshold M, the connected domain B may be determined as a false positive region, and only the connected domain A may be retained (i.e., the connected domain A may be determined as the retained connected domain); when the proportion of the area of the connected domain A to the total area of the connected domains A and B is less than or equal to the first threshold M, both the connected domains A and B may be determined as a portion of the element mask, and the connected domains A and B may be retained at the same time (i.e., the connected domains A and B may be determined as the retained connected domains).

In some embodiments, in response to determining that the count of the connected domains is greater than or equal to 3, connected domains A, B, C, ..., P may be obtained respectively according to the size of areas (S). The area of the connected domain A may be greater than the area of the connected domain B, the area of the connected domain B may be greater than an area of the connected domain C, and so on, i.e., S(A) > S(B) > S(C) > ...>S (P). Then a total area S(T) of the connected domains A, B, C, ..., P may be determined, and the connected domains may be determined. In this case, whether each connected domain (or a connected domain with an area rank before the preset rank n) is a retained connected domain may be determined in the order of the area ranks according to the corresponding preset condition. In some embodiments, in response to determining that the count of the connected domains is greater than or equal to 3, the preset condition that the largest connected domain (i.e., the connected domain A) needs to meet as the retained connected domain may relate to the relationship between the ratio of the area of the largest connected domain to the total area of the connected domains and the threshold (e.g., the first threshold M). In some embodiments, in response to determining that the count of the connected domains is greater than or equal to 3, the preset condition that the largest connected domain (i.e., the connected domain A) needs to meet as the retained connected domain may relate to a relationship between a ratio of the area of the second connected domain to the area of the largest connected domain and a threshold (e.g., a second threshold N). For example, when the ratio of the area of the connected domain A to the total area S(T) is greater than the first threshold M, i.e., S(A)/S(T) > the first threshold M, or when the ratio of the area of the connected domain B to the area of the connected domain A is less than the second threshold N, i.e., S(B)/S(A) < the second threshold N, the connected domain A may be determined as an element mask portion and retained (i.e., the connected domain A may be the retained connected domain), and the rest connected domains may be determined as the false positive regions; otherwise, the calculation may continue, i.e., it continues to determine whether the second connected domain (i.e., the connected domain B) is a retained connected domain. In some embodiments, the preset condition that the connected domain B needs to meet as the retained connected domain may relate to a relationship between a ratio of a sum of the areas of the first connected domain and the second connected domain to the total area of the connected domain and the first threshold M. In some embodiments, the preset condition that the connected domain B needs to meet as the retained connected domain may also relate to a relationship between a ratio of an area of a third connected domain to the sum of the areas of the first connected domain and the second connected domain and the threshold (e.g., the second threshold N). For example, when the ratio of the areas of the connected domain A and the connected domain B to the total area S(T) is greater than the first threshold M, i.e., S(A + B)/S(T) > the first threshold M, or when the ratio of the area of the connected domain C to the areas of the connected domain A and the connected domain B is less than the second threshold N, i.e., S(C)/S(A + B) < the second threshold N, the connected domain A and the connected domain B may be determined as the element mask portions and retained (i.e., the connected domain A and the connected domain B may be the retained connected domains), and the rest portions may be determined as the false positive regions; otherwise, the calculation may continue, i.e., it continues to determine whether the third connected domain (i.e., the connected domain C) is the retained connected domain. The determination process of the connected domain C may be similar to the determination process of the connected domain B. The preset condition that the connected domain C needs to meet as the retained connected domain may relate to a relationship between a ratio of a sum of areas of the first connected domain, the second connected domain, and the third connected domain to the total area of the connected domains and the first threshold M, or a relationship between a ratio of an area of a fourth connected domain area to the sum of the areas of the first connected domain, the second connected domain, and the third connected domain and the threshold (e.g., the second threshold N). For example, when the ratio of the areas of the connected domains A, B, and C to the total area S(T) is greater than the first threshold M, i.e., S(A + B + C)/S(T)> the first threshold M, or when the ratio of the area of the connected domain D to the areas of the connected domains A, B, and C is less than the second threshold N, i.e., S(D)/S(A + B+C) < the second threshold N, the connected domains A, B, and C may be determined as the element mask portions and retained (i.e., the connected domains A, B, and C may be the retained connected domains). Referring to the above determination process, whether the connected domains A, B, C, D,..., P, or other connected domains within the preset order n in the ranking result are the retained connected domains may be determined in sequence. It should be noted that FIG. 4 only shows the determination of whether the three connected domains are the retained connected domains. It can also be understood that the value of the preset order n in FIG. 4 is set to 4, and thus it is only necessary to determine whether the connected domains with order ranks 1, 2, and 3, i.e., the connected domain A, the connected domain B, and the connected domain C are the retained connected domains.

Finally, the retained connected domains may be output.

In some embodiments, the second threshold N may be in a range of 0.03-0.3, which ensures that the soft connected domain analysis has an expected accuracy.

As shown in FIG. 6, the left side shows a cross-sectional medical image and a stereoscopic medical image of a coarse segmentation result without soft connected domain analysis, and the right side shows a cross-sectional medical image and a stereoscopic medical image of the coarse segmentation result with the soft connected domain analysis. It can be seen that in the coarse segmentation result of the element mask based on the soft connected domain analysis, the false positive region framed by the box in the left images is removed. Compared with the conventional connected domain analysis manner, the accuracy and reliability of excluding the false positive region are higher, which directly helps to reasonably extract the bounding box of the positioning information of the element mask in the subsequent process, thereby improving the segmentation efficiency.

In some embodiments, the positioning information of the element mask may include position information of a bounding rectangle of the element mask, such as coordinate information of a border line of the bounding rectangle. In some embodiments, the bounding rectangle of the element mask may cover a positioning region of the at least one element. In some embodiments, the bounding rectangle may be displayed in the medical image in the form of a bounding rectangle frame. In some embodiments, the bounding rectangle frame corresponding to the element mask of at least one element may be constructed based on bottom edges of the connected region of the at least one element in various directions, such as the bottom edges of the connected region in up, down, left, and right directions.

In some embodiments, the bounding rectangle of the element mask may be a rectangle frame or a combination of a plurality of rectangle frames, such has a rectangle frame with a relatively large area, or a rectangle frame with a relatively large area formed by combining a plurality of rectangle frames with relatively small areas.

In some embodiments, the bounding rectangle of the element mask may be a bounding rectangle frame having only one rectangle frame. For example, when there is only one connected region (e.g., a blood vessel or an organ in the abdominal cavity), a bounding rectangle with a relatively large area may be constructed based on bottom edges of the connected region in all directions. In some embodiments, the bounding rectangle with a relatively large area may be applied to an organ having one connected region.

In some embodiments, the bounding rectangle of the element mask may be a bounding rectangle frame formed by combining a plurality of rectangle frames. For example, when there are a plurality of connected regions, the plurality of connected regions correspond to the plurality of rectangle frames, a bounding rectangle may be constructed based on bottom edges of the plurality of rectangle frames. It is understood that if the bottom edges of three rectangle frames corresponding to three connected regions construct a total bounding rectangle frame, the calculation is processed according to the total bounding rectangle frame, which reduces the amount of calculation while ensuring the expected accuracy.

In some embodiments, when the medical image includes the plurality of connected domains, the position information of the plurality of connected domains may be determined first, and then the positioning information of the element mask may be obtained based on the position information of the plurality of connected domains. For example, connected domains that meet the preset condition may be determined first, i.e., the position information of the retained connected domains may be determined, and then the positioning information of the element mask may be obtained based on the position information of the retained connected domains.

In some embodiments, in the operation 330, the determining the positioning information of the element mask may further include the following operations: positioning the element mask based on positioning coordinates of one or more preset elements.

In some embodiments, this operation 330 may be performed when the positioning of the bounding rectangle of the element mask fails. It is understood that when the coordinates of the bounding rectangle of the element mask do not exist, it is determined that the positioning of the corresponding element fails.

In some embodiments, a preset element may be selected from one or more elements with relatively stable positioning (e.g., an organ with relatively stable positioning). The probability of positioning failure may be low when positioning the preset element, thereby realizing accurate positioning of the element mask. In some embodiments, since the probability of positioning failure of the liver, the stomach, the spleen, and the kidney in the abdominal cavity is low, and the probability of the positioning failure of the lungs in the thoracic cavity is low, the positioning of the liver, the stomach, the spleen, and the kidneys may be relatively stable, and those organs may be used as the preset organs in the abdominal cavity. That is, the preset elements may include the liver, the stomach, the spleen, the kidneys, the lungs, or any other feasible organ tissue. In some embodiments, the organ mask in the abdominal cavity may be repositioned based on the positioning coordinates of the liver, the stomach, the spleen, and the kidneys. In some embodiments, the organ mask in the thoracic cavity may be positioned based on the positioning coordinates of the lungs.

In some embodiments, the element mask may be repositioned with the positioning coordinates of the preset elements as reference coordinates. In some embodiments, when an element that fails to be positioned is located in the abdominal cavity, the positioning coordinates of the liver, the stomach, the spleen, and the kidney may be used as the coordinates for repositioning, and the element that fails to be positioned in the abdominal cavity may be repositioned accordingly. In some embodiments, when the element that fails to be positioned is located in the thoracic cavity, the positioning coordinates of the lungs may be used as the coordinates for repositioning, and the element that fails to be positioned in the thoracic cavity may be repositioned accordingly. Merely by way of example, when the element that fails to be positioned is located in the abdominal cavity, positioning coordinates of a liver top, a kidney bottom, a left portion of the spleen, and a right portion of the liver may be used as coordinates of cross-sectional defense lines (an upper side and a lower side) and a coronal plane direction (a left side and a right side) for repositioning, and a frontmost end and a rearmost end of the coordinates of the liver top, the kidney bottom, the left portion of the spleen, and the right portion of the liver taken may be taken as coordinates of a newly positioned sagittal plane direction (a front side and a rear side), and the element that fails to be positioned in the abdominal cavity may be repositioned accordingly. Merely by way of example, when the element that fails to be positioned is located in the thoracic cavity, a bounding rectangle frame formed by the positioning coordinates of the lungs may be expanded by a certain pixel, and the element that fails to be positioned in the thoracic cavity may be repositioned accordingly.

The element mask is accurately positioned based on the positioning coordinates of the preset elements, which can improve the segmentation accuracy and reduce the segmentation time, thereby improving the segmentation efficiency, while reducing the segmentation calculation amount and saving memory resources.

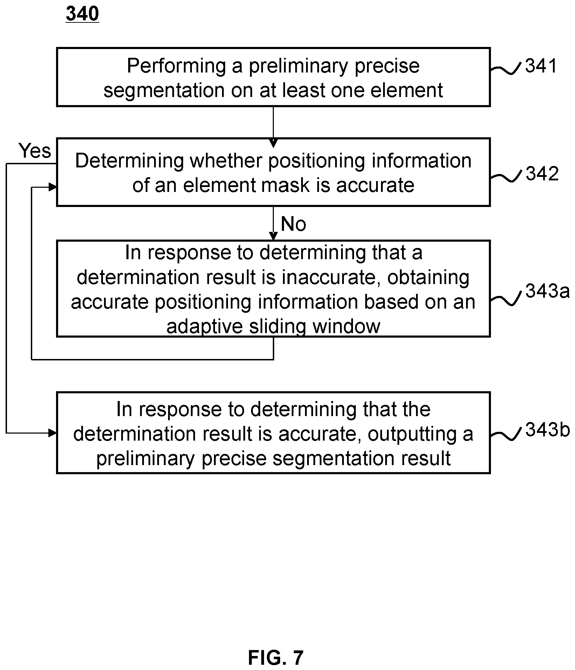

FIG. 7 is a flowchart illustrating an exemplary process of precise segmentation on at least one element according to some embodiments of the present disclosure.

In some embodiments, in the operation 340, the performing the precise segmentation on the at least one element based on the positioning information of the mask may include the following sub-operations.

In 341, a preliminary precise segmentation may be performed on the at least one element. The preliminary precise segmentation may be a precise segmentation performed based on positioning information of an element mask obtained by a coarse segmentation. In some embodiments, the preliminary precise segmentation may be performed on the at least one element based on input data and a bounding rectangle frame positioned by the coarse segmentation. The element mask of the precise segmentation may be generated by the preliminary precise segmentation.

In 342, whether the positioning information of the element mask is accurate may be determined. With the operation 342, whether the positioning information of the element mask obtained by the coarse segmentation is accurate may be determined, and whether the coarse segmentation is accurate may be determined.

In some embodiments, the positioning information of the element mask may be obtained by analyzing the element mask obtained by the preliminary precise segmentation, and the positioning information of the coarse segmentation may be compared with the positioning information of the precise segmentation. In some embodiments, the bounding rectangle frame of the element mask obtained by the coarse segmentation may be compared with the bounding rectangle frame of the element mask obtained by the precise segmentation to determine a difference between these bounding rectangle frames. In some embodiments, the bounding rectangle frame of the element mask obtained by the coarse segmentation may be compared with the bounding rectangle frame of the element mask obtained by the precise segmentation in six directions in a 3D space (i.e., the entire bounding rectangle frame may be a cube in the 3D space) to determine the difference between these bounding rectangle frames. Merely by way of example, an overlap rate between each side of the bounding rectangle frame of the element mask obtained by the coarse segmentation and each side of the bounding rectangle frame of the element mask obtained by the precise segmentation may be determined, or difference values between six vertex coordinates of the bounding rectangle frame of the element mask obtained by the coarse segmentation and six vertex coordinates of the bounding rectangle frame of the element mask obtained by the precise segmentation may be determined.

In some embodiments, whether the positioning information of the element mask obtained by the coarse segmentation is accurate may be determined according to the positioning information of the element mask obtained by the preliminary precise segmentation. In some embodiments, whether a determination result is accurate may be determined according to a difference between the positioning information of the coarse segmentation and the positioning information of the precise segmentation. In some embodiments, the positioning information may be the bounding rectangle frame of the element mask, and whether the bounding rectangle of the element mask obtained by the coarse segmentation is accurate may be determined according to the bounding rectangle frame of the element mask obtained by the coarse segmentation and the bounding rectangle frame of the element mask obtained by the precise segmentation. In this case, the difference between the positioning information of the coarse segmentation and the positioning information of the precise segmentation refers to a distance between the closest border lines in the bounding rectangle frame of the coarse segmentation and the bounding rectangle frame of the precise segmentation. In some embodiments, when the difference between the positioning information of the coarse segmentation and the positioning information of the precise segmentation is large (i.e., the distance between the closest border lines in the bounding rectangle frame of the coarse segmentation and the bounding rectangle frame of the precise segmentation is large), it is determined that the positioning information of the coarse segmentation is accurate. When the difference between the positioning information of the coarse segmentation and the positioning information of the precise segmentation is small (i.e., the distance between the closest border lines in the bounding rectangle frame of the coarse segmentation and the bounding rectangle frame of the precise segmentation is small), it is determined that the positioning information of the coarse segmentation is inaccurate. It should be noted that the bounding rectangle frame of the coarse segmentation may be obtained by expanding (e.g., expanding 15-20 voxels) pixels on border lines of original close elements of the coarse segmentation. In some embodiments, whether the positioning information of the coarse segmentation is accurate may be determined based on a relationship between the distance between the closest border lines in the bounding rectangle frame of the coarse segmentation and the bounding rectangle frame of the precise segmentation and a preset threshold. For example, when the distance is less than the preset threshold, it is determined that the positioning information of the coarse segmentation is inaccurate; and when the distance is greater than the preset threshold, it is determined that the positioning information of the coarse segmentation is accurate. In some embodiments, in order to ensure the determination accuracy, a value range of the preset threshold may be less than or equal to 5 voxels.

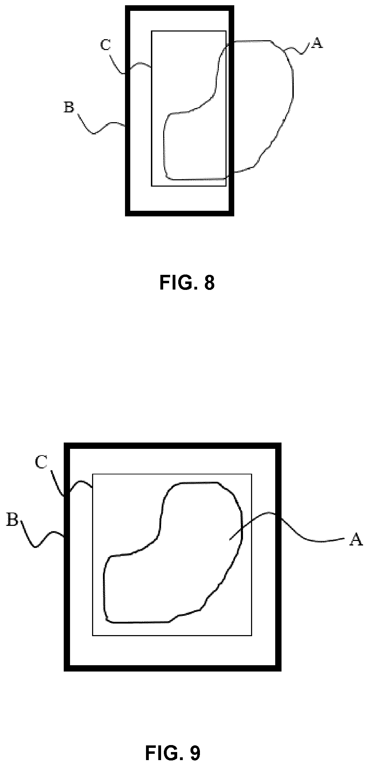

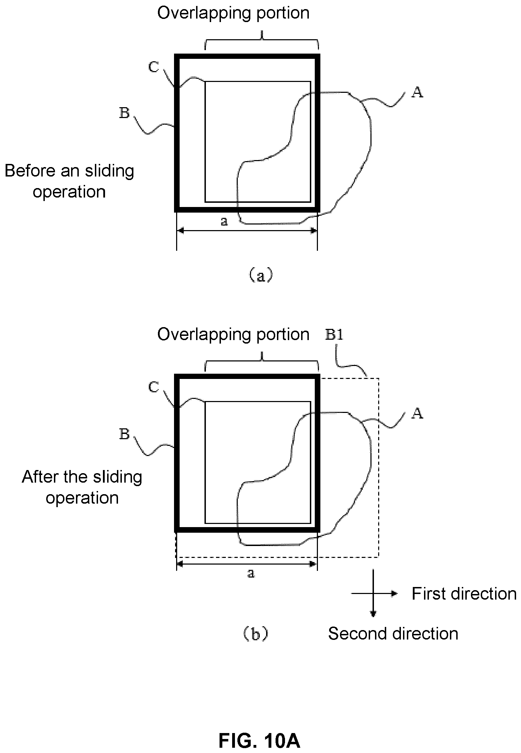

FIGs. 8-9 are schematic diagrams illustrating an exemplary process of determining positioning information of an element mask according to some embodiments of the present disclosure. FIG. 10A is a schematic diagram illustrating an exemplary process of determining a sliding direction based on positioning information of an element mask according to some embodiments of the present disclosure.

FIG. 8 and FIG. 9 show an element mask A obtained by a coarse segmentation and a bounding rectangle frame B of the element mask A (i.e., positioning information of the element mask A), and a bounding rectangle frame C after a preliminary precise segmentation based on a bounding rectangle frame of a coarse segmentation. FIG. 10A also shows a sliding window B1 obtained after the bounding rectangle frame B of the coarse segmentation slides, wherein (a) is a schematic diagram before a sliding operation, and (b) is a schematic diagram after the sliding operation. In addition, for convenience, a plane rectangle frame in a plane of a 3D bounding rectangle frame is used as an example for illustration. It can be understood that there are five other plane rectangle frames in the 3D bounding rectangle frame. That is, when the specific calculation of the 3D bounding rectangle frame is performed, there are border lines in six directions. Here, only four border lines of a certain plane may be used for illustration.

Merely by way of example, as shown in FIG. 8, a difference between a right side border line in the bounding rectangle frame C of the precise segmentation and a border line corresponding to the bounding rectangle frame B of the coarse segmentation is small. It can be determined that a direction corresponding to the right side of the bounding rectangle frame B of the coarse segmentation is inaccurate, and the right side border line needs to be adjusted. However, differences between upper, lower and left border lines in C and upper, lower, and left sides in B are large. It can be determined that directions corresponding to the upper, lower, and left sides of the bounding rectangle frame B of the coarse segmentation are accurate. Merely by way of example, as shown in FIG. 9, differences between border lines of four sides in the bounding rectangle frame C of the precise segmentation and the border lines corresponding to the bounding rectangle frame B of the coarse segmentation are large. It can be determined that the border lines of the four sides in the bounding rectangle frame B of the coarse segmentation are all accurate. It should be noted that there are six directions for the element mask A, and only four border lines are used for illustration in FIG. 8 and FIG. 9. In an actual situation, twelve border lines of the six directions in the element mask A may be determined.

In 343a, when the determination result is inaccurate, accurate positioning information may be obtained based on an adaptive sliding window. In some embodiments, when a coarse segmentation result is inaccurate, the elements obtained by the precise segmentation may be inaccurate. Accurate positioning information may be obtained by performing the corresponding adaptive sliding window calculation to continue the precise segmentation.

In some embodiments, obtaining the accurate positioning information based on the adaptive sliding window may include: determining at least one direction in which positioning information is inaccurate; and performing the adaptive sliding window calculation in the at least one direction according to an overlap rate parameter. In some embodiments, at least one direction in which the bounding rectangle frame is inaccurate may be determined; after determining that the bounding rectangle frame of the coarse segmentation is inaccurate, the bounding rectangle frame of the coarse segmentation may be slid in the corresponding direction according to the input preset overlap rate parameter, i.e., a sliding window operation may be performed, and the sliding window operation may be repeated until all bounding rectangles are completely accurate. The overlap rate parameter refers to a ratio of an area of an overlapping region between an initial bounding rectangle frame and a bounding rectangle frame after sliding to an area of the initial bounding rectangle frame. When the overlap rate parameter is high, a sliding step length of the sliding window operation may be relatively short. In some embodiments, in order to ensure that the process of the sliding window calculation is more concise (i.e., the operation of the sliding window operation is fewer), the overlap rate parameter may be set to a relatively small value. In order to ensure that the result of the sliding window calculation is more accurate, the overlap rate parameter may be set to a relatively large value. In some embodiments, the sliding step length of the sliding window operation may be determined according to a current overlap rate parameter. According to the determination process of FIG. 8, it can be seen that the directions corresponding to the right and lower border lines of the bounding rectangle frame B of the coarse segmentation in FIG. 10 A are inaccurate. For the convenience of description, the direction corresponding to the right border line of the bounding rectangle frame B may be denoted as a first direction (the first direction may be perpendicular to the right border line of B), and the direction corresponding to the lower border line may be denoted as a second direction (the second direction may be perpendicular to the lower border line of B). Merely by way of example, as shown in FIG. 10A, assuming that the length of the bounding rectangle frame B is a, when the overlap rate parameter is 60%, the corresponding step length is determined as a*(1-60%). As mentioned above, the right border line of the bounding rectangle frame B can slide a*(1- 60%) along the first direction. Similarly, the lower border line of the bounding rectangle frame B may slide along the second direction by a corresponding step length. The right border line and the lower border line of the bounding rectangle frame B may repeat the corresponding sliding window operation, respectively, until the bounding rectangle frame B is completely accurate, such as a sliding window B1 shown in FIG. 10A(b). Referring to FIG. 8 and FIG. 10A, when it is determined that the bounding rectangle frame (i.e., the positioning information of a target structure mask) of the coarse segmentation is inaccurate, coordinate values of the border lines in the six directions of the bounding rectangle of the fine segmentation may be compared with coordinate values of the border lines in the six directions of the bounding rectangle of the coarse segmentation one by one. For each of the six directions, when a difference value between the coordinate value of the border line in the direction of the bounding rectangle frame of the fine segmentation and the coordinate value of the border line in the direction of the bounding rectangle frame of the coarse segmentation is less than a coordinate difference threshold (e.g., the coordinate difference threshold is 5 pt), , it can be determined that the border lines of the bounding rectangle frames are in an inaccurate direction. The coordinate difference threshold may be set according to an actual situation, which is not limited here.

As another example, as shown in FIG. 8, the pixel coordinates in the four directions corresponding to the four sides of the bounding rectangle frame C of the precise segmentation may be compared with the pixel coordinates in the four directions corresponding to the four border lines in the bounding rectangle frame B of the coarse segmentation one by one. When a difference in between the pixel coordinates in one of the four directions is less than a coordinate difference threshold of 8 pt, it is determined that the direction of the bounding rectangle frame of the coarse segmentation in FIG. 8 is inaccurate. For example, if a difference between the pixel coordinates in the direction corresponding to upper sides is 20 pt, a difference between the pixel coordinates in the direction corresponding to lower sides is 30 pt, a difference between the pixel coordinates in the direction corresponding to right sides is 1 pt, and a difference between the pixel coordinates in the direction corresponding to left sides is 50 pt, the direction corresponding to the right sides may be inaccurate, and the directions corresponding to the upper sides, the lower sides, and the left sides may be accurate.

As another example, referring to FIG. 10A, B1 is a bounding rectangle frame (also referred to as a sliding window) obtained after the bounding rectangle frame B of the coarse segmentation slides. It can be understood that the sliding window is a bounding rectangle frame of the coarse segmentation that meets the expected accuracy standard, and the border lines (e.g., a right border line, and a lower border line) of the bounding rectangle frame B of the coarse segmentation need to be slid along the corresponding directions (e.g., a first direction, and a second direction) to the position of the sliding window B1 by corresponding step lengths. Directions corresponding to the border lines that do not meet the standard may be moved in sequence. For example, the right border line of B may be slid first, then the lower border line of B may be slid to a specified position of the sliding window, and the directions corresponding to the left side and the upper side of B may be standard and may not be slid. It can be understood that a step length of each side sliding depends on an overlap rate of B1 and B. The overlap rate refers to a ratio of a current overlapping region of the bounding rectangle frame B of the coarse segmentation and the sliding window B1 to a total area. For example, the current overlap rate may be 40 %, etc. It should be noted that the sliding order of the border lines of the bounding rectangle frame B of the coarse segmentation may be from left to right, from top to bottom, or other feasible orders, which is not limited here.

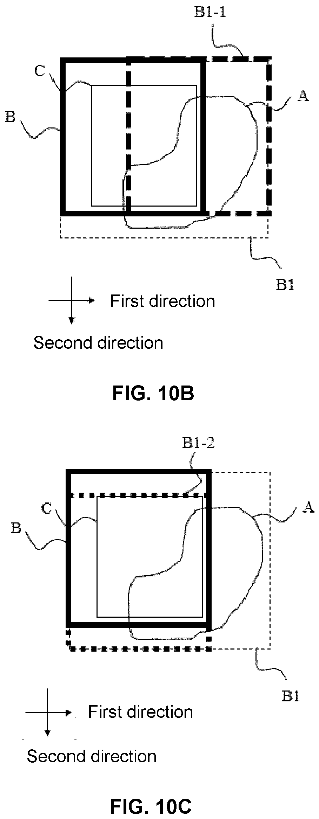

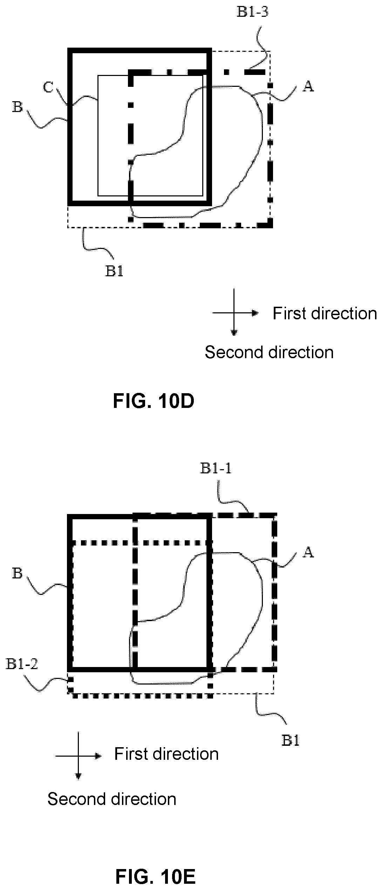

FIGs. 10B-10E are schematic diagrams illustrating an exemplary process of performing a precise segmentation after sliding window according to some embodiments of the present disclosure. Referring to FIGs. 10B-10E, in some embodiments, an accurate bounding rectangle frame of a coarse segmentation may be obtained by performing adaptive window sliding based on an original bounding rectangle frame (e.g., an original sliding window) of the coarse segmentation, and accurate coordinate values of the bounding rectangle frame may be obtained. A precise segmentation may be performed on a new sliding window based on the coordinate values and an overlap rate parameter, and a precise segmentation result may be superimposed with a preliminary precise segmentation result to obtain a final precise segmentation result. Specifically, referring to FIG. 10B, a sliding window B1 (a bounding rectangle frame of a maximum range after a sliding window operation) may be obtained by performing the sliding window operation on an original sliding window B, a sliding window B1-1 may be obtained by sliding B along a first direction by a corresponding step length, and a precise segmentation result of the sliding window B1-1 may be obtained by performing a precise segmentation on an entire domain range of the sliding window B1-1. Further, referring to FIG. 10C, a sliding window B1-2 may be obtained by sliding B along a second direction by a corresponding step length, and a precise segmentation result of the sliding window B1-2 may be obtained by performing a precise segmentation on an entire domain range of the sliding window B1-2. Further, referring to FIG. 10D, a sliding window B1-3 (e.g., the sliding window B1-2 may be obtained by performing a sliding operation on B described in FIG. 10C, and then the sliding window B1-3 may be obtained by sliding the sliding window B1-2) may be obtained by sliding B, and a precise segmentation result of the sliding window B1-3 may be obtained by performing a precise segmentation on an entire domain range of the sliding window B1-3. The precise segmentation results of the sliding window B1-1, the sliding window B1-2, and the sliding window B1-3 may be superimposed with preliminary precise segmentation results to obtain final precise segmentation results. It should be noted that the sizes of the sliding window B1-1, the sliding window B1-2, and the sliding window B1-3 may be the same as the size of B. The sliding window B1 may be a final sliding window result obtained by performing continuous sliding window operations on the original sliding window B, i.e., the sliding window B1-1, the sliding window B1-2, and sliding window B1-3. In some embodiments, when the precise segmentation results of the sliding window B1-1, the sliding window B1-2, and the sliding window B1-3 are superimposed with the preliminary precise segmentation results, a repeated superimposed portion may exist. For example, in FIG. 10E, the sliding window B1-1 and the sliding window B1-2 may have an intersection. When the segmentation results are superimposed, the intersection may be repeatedly superimposed. For this situation, the following process may be adopted. For a portion of an element mask A, if a segmentation result of one sliding window for this portion is accurate, and a segmentation result of another sliding window is inaccurate, the segmentation result of the sliding window with the accurate segmentation result may be used as the segmentation result of the portion; if the segmentation results of both sliding windows are accurate, the segmentation result of the right sliding window may be used as the segmentation result of the portion; if the segmentation results of both sliding windows are inaccurate, the segmentation result of the right sliding window may be used as the segmentation result of the portion, and the precise segmentation may be continued until the segmentation result is accurate.

In some embodiments, as shown in FIG. 7, when the determination result is inaccurate, obtaining accurate positioning information based on the adaptive sliding window may be a cyclic process. Specifically, after the border lines of the precise segmentation and the border lines of the coarse segmentation are compared, updated coordinate values of the bounding rectangle frame of the precise segmentation may be obtained through the adaptive sliding window. The bounding rectangle frame of the precise segmentation may be expanded by a certain count of pixels and set as a bounding rectangle frame of a coarse segmentation of a new cycle. Then a new bounding rectangle frame of a precise segmentation may be obtained by performing the precise segmentation on the new bounding rectangle frame, and whether the new bounding rectangle frame of the precise segmentation meets the accuracy requirements may be determined. If the accuracy requirements are met, the cycle may be ended; if the accuracy requirements are not met, the cycle may continue. In some embodiments, the precise segmentation may be performed on at least one element in the coarse segmentation using a deep convolutional neural network model. In some embodiments, historical medical images preliminarily obtained before the coarse segmentation may be used as training data, and the deep convolutional neural network model may be trained with historical precise segmentation result data. In some embodiments, the historical medical images and the historical precise segmentation result data may be obtained from historical scan medical images and historical precise segmentation result data of a scan subject from the medical scanning device 110. In some embodiments, the historical scan medical images and the historical precise segmentation result data of the scan subject may be obtained from the one or more terminals 130, the processing device 140, and the storage device 150.

In 343b, when the determination result is accurate, a preliminary precise segmentation result may be output.

In some embodiments, when the determination result (i.e., the coarse segmentation result) is accurate, it is determined that the positioning information of the at least one element obtained by performing the precise segmentation based on the coarse segmentation result is accurate, and the preliminary precise segmentation result may be output.

In some embodiments, at least one element result data of the precise segmentation may be output. In some embodiments, in order to further reduce noise and optimize the image display effect, an image post-processing operation may be performed before the segmentation result is output. The image post-processing operations may include edge smoothing and/or denoising of the image. In some embodiments, the edge smoothing may include smoothing or blurring to reduce noise or distortion of the medical image. In some embodiments, the smoothing or the blurring may be performed in the following ways: mean filtering, median filtering, Gaussian filtering, and bilateral filtering.

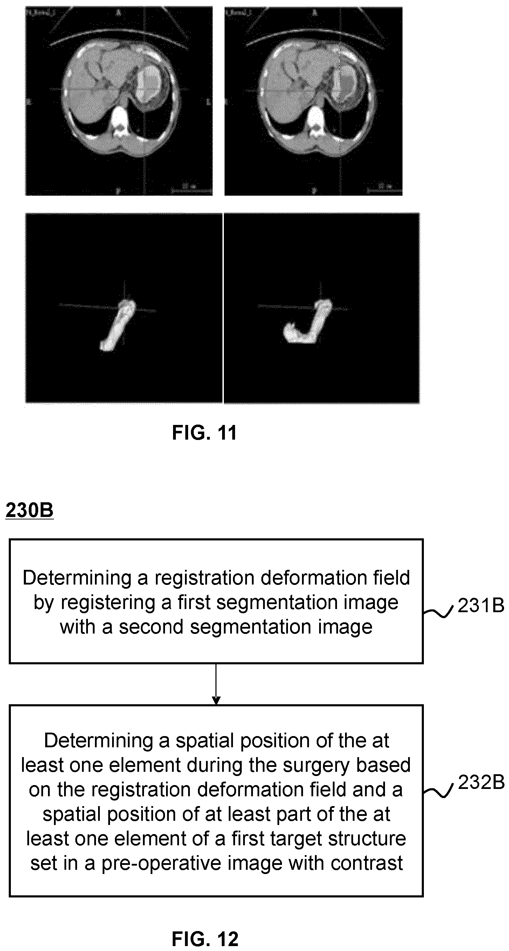

FIG. 11 is a comparison diagram illustrating exemplary effects of segmentation results according to some embodiments of the present disclosure.

As shown in FIG. 11, the left side shows a cross-sectional medical image and a stereoscopic medical image of a coarse segmentation result using the traditional technology, and the right side shows a cross-sectional medical image and a stereoscopic medical images using an organ segmentation manner provided by the embodiments of the present disclosure. It can be seen that a target organ segmentation result displayed in a segmentation result image on the right side is more complete than a target organ segmentation result displayed in a segmentation result image on the left side, which reduces the risk of missing segmented organs, improves the segmentation accuracy, and ultimately improves the overall segmentation efficiency.

In 230B, a spatial position of a third target structure set during the surgery may be determined by registering a first segmentation image with a second segmentation image. In some embodiments, the operation 230B may be performed by the result determination module 2330.

The third target structure set refers to a structure set obtained after the first segmentation image is registered with the second segmentation image. In some embodiments, the third target structure set may include a target organ, blood vessels and a lesion in the target organ, and other regions/organs (e.g., one or more non-interventional regions, and all important organs). In some embodiments, in a fast segmentation mode, the other regions/organs refer to the one or more non-interventional regions. In a precise segmentation mode, the other regions/organs refer to all the important organs. In some embodiments, at least one element in the third target structure set may be included in the first target structure set, and at least one element in the third target structure set may not be included in the second target structure set. For example, when the first target structure set includes the target organ and the blood vessels and the lesion in target organ, and the second target structure set includes the one or more non-interventional regions (or all the important organs), the target organ, and the lesion, the blood vessels in the target organs may be included in the first target structure set and may be not included in the second target structure set. In some embodiments, a fourth target structure set may also be regarded as a portion of the third target structure set, such as the one or more non-interventional regions, all the important organs outside the target organ.

The third target structure set can more comprehensively and accurately reflect a current condition of a scan subject (e.g., a patient). Path planning of the interventional surgery may be performed based on a spatial position of the third target structure set, such that a puncture needle can effectively avoid one or more non-interventional regions and/or all the important organs to successfully reach the lesion.

In some embodiments, the first segmentation image may include precise structural features (e.g., blood vessels in a target organ, the target organ, a lesion) of the first target structure set; the second segmentation image may include precise structural features (e.g., a target organ, a lesion, one or more non-interventional regions/all the important organs) of the second target structure set. In some embodiments, before registration, a separation processing of appearance features of the target structure set and a background may be performed on the first segmentation image and the second segmentation image. In some embodiments, the separation processing of the appearance features and the background may be performed based on an artificial neural network (e.g., a linear decision function, etc.), a threshold-based segmentation manner, an edge-based segmentation manner, a cluster analysis-based image segmentation manner (e.g., K-means, etc.), or any other feasible algorithm, such as a wavelet transform-based segmentation manner, etc.

The registration process is described below with reference to an example of the first segmentation image including the blood vessels in the target organ (e.g., the target organ) and the structural features of the target organ (i.e., the first target structure set includes the blood vessels in the target organ and the target organ), and the second segmentation image including the structural features of the target organ, the lesion, and the one or more non-interventional region/all the important organs (i.e., the second target structure set includes the target organ, the lesion, and the one or more non-interventional regions/all the important organs). It is understood that the structural features of the lesion are not limited to being included in the second segmentation image. In some embodiments, the structural features of the lesion may also be included in the first segmentation image, or the structural features of the lesion may be included in both the first segmentation image and the second segmentation image.

FIG. 12 is a flowchart illustrating an exemplary process of registering a first segmentation image with a second segmentation image according to some embodiments of the present disclosure. In 231B, a registration deformation field may be determined by registering the first segmentation image with the second segmentation image.

Registration refers to an image processing operation that makes corresponding points of the first segmentation image and the second segmentation image have consistent spatial positions and anatomical positions through spatial transformation. The registration deformation field may reflect a spatial position change between the first segmentation image and the second segmentation image. In some embodiments, after registration, an intra-operative image without contrast may be transformed based on the registration deformation field, such that the transformed intra-operative image without contrast may be completely consistent with a pre-operative image with contrast in the spatial position and the anatomical position.

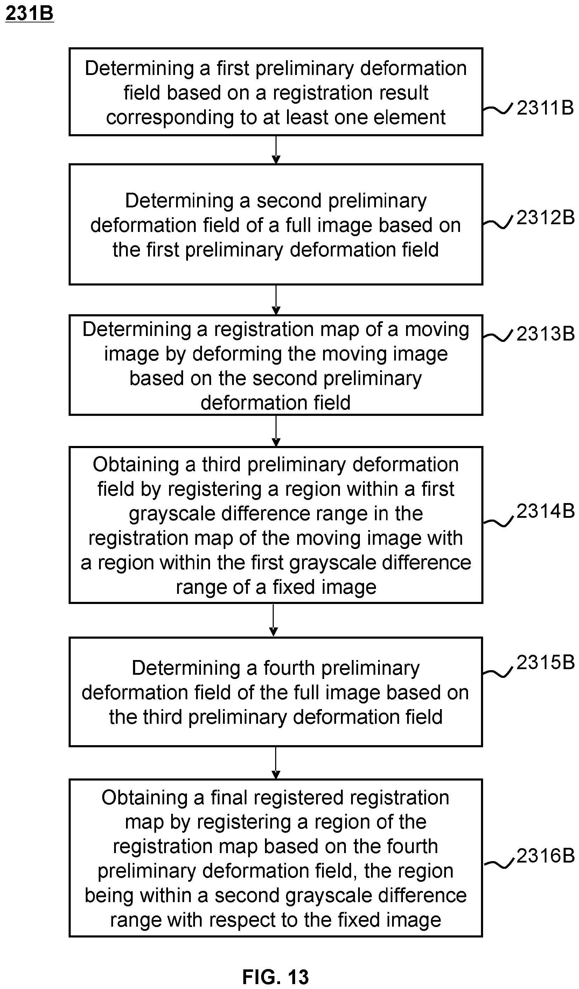

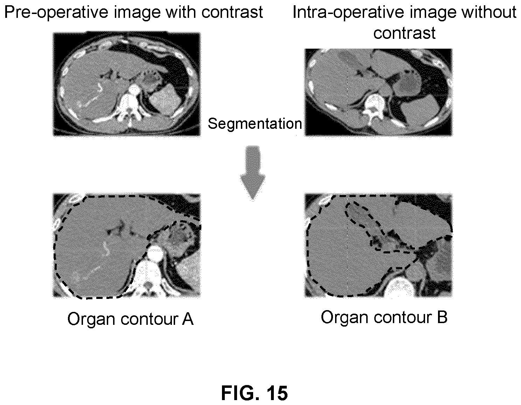

FIGs. 13-14 are flowcharts illustrating an exemplary process of determining a registration deformation field according to some embodiments of the present disclosure. FIG. 15 is a schematic diagram illustrating an exemplary process of obtaining a first segmentation image and a second segmentation image by segmentation according to some embodiments of the present disclosure.

In some embodiments, in the operation 231B, the determining the registration deformation field by registering the first segmentation image with the second segmentation image may include the following sub-operations.

In 2311B, a first preliminary deformation field may be determined based on a registration result corresponding to at least one element.

In some embodiments, the at least one element may include an element contour (e.g., an organ contour, a blood vessel contour, and a lesion contour) of the first segmentation image and the second segmentation image.

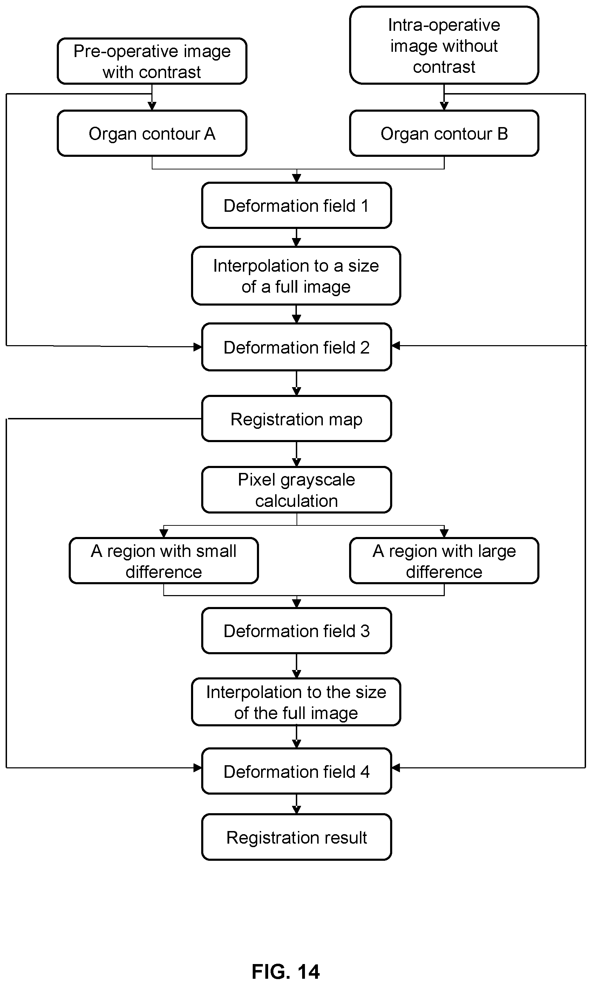

The registration corresponding to the at least one element refers to a registration between image regions covered by the element contour (e.g., a mask). For example, referring to FIGs. 14 and 15, registration is performed between an image region covered by an organ contour A of a target organ and an image region covered by an organ contour B of the target organ. The image region covered by the organ contour A is a region with the same or substantially the same grayscale in a dotted region in the lower left image in FIG. 15 obtained after segmenting the pre-operative image with contrast. The image region covered by the organ contour B is a region with the same or substantially the same grayscale in a dotted region in the lower right image in FIG. 15 obtained after segmenting the intra-operative image without contrast.

In some embodiments, the first preliminary deformation field (e.g., a deformation field 1 in FIG. 14) may be obtained by a regional registration of the image region covered by the organ contour A with the image region covered by the organ contour B. In some embodiments, the first preliminary deformation field may be a local deformation field. For example, a local deformation field of a liver contour may be obtained by using a pre-operative contour A and an intra-operative contour B of the liver.

In 2312B, a second preliminary deformation field of a full image may be determined based on the first preliminary deformation field, the full image including the at least part of the at least one element.

The full image refers to an image of a region (e.g., a region of the human body) including at least part of the at least one element. For example, when the target organ is the liver, the full image may be an image of an entire abdominal cavity. As another example, when the target organ is a lung, the full image may be an image of an entire thoracic cavity.

In some embodiments, the second preliminary deformation field of the full image may be determined by performing interpolation on the first preliminary deformation field. In some embodiments, the second preliminary deformation field may be a global deformation field. For example, in FIG. 14, a deformation field 2 with an entire image size may be determined by performing interpolation on the deformation field 1.

In 2313B, a registration map of a moving image may be determined by deforming the moving image based on the second preliminary deformation field.

The moving image refers to an image to be registered, such as the pre-operative image with contrast or the intra-operative image without contrast. For example, when the intra-operative image without contrast is registered to the pre-operative scan image, the moving image may be the intra-operative image without contrast. The intra-operative image without contrast may be registered by the registration deformation field so as to make the spatial position of the intra-operative image without contrast consistent with that of the pre-operative scan image. As another example, when the pre-operative image with contrast is registered to the intra-operative image without contrast, the moving image may be the pre-operative image with contrast. The pre-operative scan image may be registered by the registration deformation field so as to make the spatial position of the pre-operative scan image consistent with that of the intra-operative image without contrast. The registration map of the moving image may be an image of an intermediate registration result obtained during the registration process. Taking the registration of the pre-operative image with contrast to the intra-operative image without contrast as an example, the registration map of the moving image may be an intermediate intra-operative image without contrast obtained during the registration process. For ease of understanding, the registration process is described in detail in the embodiments of the present disclosure with reference to the registration of the pre-operative image with contrast to the intra-operative image without contrast as an example.

In some embodiments, as shown in FIG. 14, the registration map of the pre-operative image with contrast, i.e., the intermediate registration result of the intra-operative image without contrast, may be determined by deforming the moving image (i.e., the pre-operative image with contrast) based on the obtained deformation field 2 of the full image. For example, as shown in FIG. 14, the registration map may be obtained by deforming the pre-operative image with contrast (e.g., an abdominal image with contrast) based on the obtained deformation field of the abdominal cavity where the liver is located.

In 2314B, a third preliminary deformation field may be obtained by registering a region within a first grayscale difference range in the registration map of the moving image with a region within the first grayscale difference range of a fixed image.

In some embodiments, the fixed image refers to a target image before registration, and is also referred to as a target image that is not registered. For example, when the pre-operative image with contrast is registered to the intra-operative image without contrast, the fixed image may be the intra-operative image without contrast that is not registered. In some embodiments, the third preliminary deformation field may be a local deformation field. In some embodiments, the operation 2314B may include the following operations: performing a pixel grayscale calculation on different regions in the registration map and the corresponding regions in the fixed image to obtain corresponding grayscale values; for each region in the registration map of the floating map, determining a difference value between a grayscale value of the region and a grayscale value of the corresponding region in the fixed image; when the difference value is within the first grayscale difference range, obtaining the third preliminary deformation field by elastically registering the region in the registration map with the corresponding region of the fixed image. In some embodiments, when the difference value is within the first grayscale difference range, it indicates that a difference between the region of the registration map and the corresponding region of the fixed image is not large or is relatively small. For example, when the first grayscale difference range is in a range of 0-150, a grayscale difference between a region Q1 of the registration map and the same region of the fixed image is 60, and a grayscale difference between a region Q2 of the registration map and the same region of the fixed image is 180, a difference between the regions Q1 of the two images (i.e., the registration map and the fixed image) is not large, while the difference between the regions Q2 is large, and only the regions Q1 of the two images are registered. In some embodiments, as shown in FIG. 14, a deformation field 3 (i.e., a third preliminary deformation field) may be obtained by elastically registering the region within the first grayscale difference range in the registration map with the region (e.g., the region with not too large difference) within the first grayscale difference range of the fixed image.

In 2315B, a fourth preliminary deformation field of the full image may be determined based on the third preliminary deformation field.

In some embodiments, the fourth preliminary deformation field of the full image may be obtained by performing interpolation on the third preliminary deformation field. In some embodiments, the fourth preliminary deformation field may be a global deformation field. In some embodiments, a global fourth preliminary deformation field may be obtained from a local third preliminary deformation field through the operation 2315B. For example, in FIG. 14, a deformation field 4 with a full image size may be determined by performing interpolation on the deformation field 3.

In 2316B, a final registered registration map may be obtained by registering a region of the registration map based on the fourth preliminary deformation field, the region being within a second grayscale difference range with respect to the fixed image.

In some embodiments, the region within the second grayscale difference range may be a region where a difference value of the grayscale values is large based on the comparison of the grayscale values of the registration map with the grayscale values of the fixed image. In some embodiments, a grayscale difference threshold (e.g., the grayscale difference threshold may be 150) may be set. A region where a difference between the grayscale values of the registration map and the grayscale values of the fixed image is less than the grayscale difference threshold may be a region within the first grayscale difference range, and a region where the difference between the grayscale values of the registration map and the grayscale values of the fixed image is greater than the grayscale difference threshold may be a region within the second grayscale difference range.

In some embodiments, the final registered registration map may be a final image that has consistent spatial position and anatomical position with the intra-operative image without contrast generated by performing multiple deformations on the moving image (e.g., the pre-operative image with contrast) based on at least one deformation field. In some embodiments, as shown in FIG. 6, the final registered registration map may be obtained by registering the region within the second grayscale difference range (i.e., the grayscale difference is relatively large) based on the fourth preliminary deformation field. For example, for a region outside the spleen with a relatively large grayscale value difference, a final registration map may be obtained by deforming the region based on the deformation field 4.

In some embodiments, one or more elements that are segmented in the moving image but not segmented in the fixed image (e.g., the blood vessels in the target organ) may be mapped from the moving image to the fixed image using the registration manner described in FIGs. 13-14. For example, the moving image may be the pre-operative image with contrast and the fixed image may be the intra-operative image without contrast, the blood vessels in the target organ may be segmented in the pre-operative image with contrast but not segmented in the intra-operative image without contrast. The blood vessels in the target organ may be mapped to the intra-operative image without contrast by registration. It can be understood that the registration manner of FIGs. 13-14 can also be applied to the registration of the one or more non-interventional regions in the fast segmentation mode and all the important organs in the precise segmentation mode, or similar effects can be achieved only by the corresponding segmentation manner.

In 232B, a spatial position of the at least one element during the surgery may be determined based on the registration deformation field and a spatial position of the at least part of the at least one element of the first target structure set in the pre-operative image with contrast. In some embodiments, the spatial positions of the blood vessels in the target organ (hereinafter referred to as the blood vessels) during the surgery may be determined based on the registration deformation field and the blood vessels in the target organ in the pre-operative image with contrast.

In some embodiments, the spatial positions of the blood vessels during the surgery may be determined based on the registration deformation field and the blood vessels in the pre-operative image with contrast based on the following formula (1):

$$\tilde{I}(x,y,z) = I_Q((x,y,z) + u(x,y,z)), (1)$$

where $I_Q$ denotes the pre-operative image with contrast, (x,y,z) denotes 3D spatial coordinates of the blood vessels, $u(x, y, z)$ denotes the registration deformation field from the pre-operative image with contrast to the intra-operative image without contrast, and $\tilde{I}(x,y,z)$ denotes the spatial positions of the blood vessels in the intra-operative image without contrast. In some embodiments, $u(x,y,z)$ can also be understood as an offset of the 3D coordinates of the elements (e.g., the blood vessels in the target organ) in the moving image to the 3D coordinates in the final registered registration map.

Accordingly, the blood vessels in the pre-operative image with contrast may be deformed to positions that are consistent with their positions during the surgery based on the registration deformation field determined in the operation 232B.

In some embodiments, a center point of the lesion may be determined based on the spatial positions of the determined intra-operative blood vessels and the lesion (included in the second segmentation image of the intra-operative image without contrast), and a safety region around the lesion and a potential needle insertion region may be determined. In some embodiments, the safety region around the lesion and the potential needle insertion region may be determined based on the determined one or more interventional regions and non-interventional regions. In some embodiments, a reference path from a percutaneous needle insertion point to the center point of the lesion may be planned based on the potential needle insertion region and basic obstacle avoidance constraints. In some embodiments, the basic obstacle avoidance constraints may include a needle insertion angle of the path, a needle insertion depth of the path, a non-intersection between the path, the blood vessels, and the important organs, etc.

It should be noted that the above description of the process 200B and the process 300 is only for example and explanation, and does not limit the scope of application of the present disclosure. For those skilled in the art, various modifications and changes can be made to the process 200B and the process 300 under the guidance of the present disclosure.

In some embodiments, in the process of obtaining the one or more non-interventional regions by segmenting the intra-operative image without contrast in the fast segmentation mode, it is necessary to fuse segmentation results of one or more non-interventional regions (e.g., part of a thoracic and abdominal region after removing a fat region, i.e., a thoracic-abdominal fat-free region) and the target organ. In this process, a non-interventional region mask may be attached to a surface of a target organ mask, which affects the planning of a puncture path and causes the emergence of a large number of connected domains. In this case, a mask fusion manner based on a fast segmentation is required, which can solve the problem of too many non-interventional region masks and connected domains attached to the surface of the target organ mask without changing an original mask of the target organ and main masks of the one or more non-interventional regions, and finally obtain an accurate fast segmentation result for puncture planning. More descriptions may be found below.

FIG. 16 is a flowchart illustrating an exemplary method for an image-assisted interventional surgery according to some embodiments of the present disclosure. As shown in FIG. 16, a process 1600 may include the following operations.

In 1610, a fat-free mask may be obtained by segmenting a target structure set from a medical image. In some embodiments, the operation 1610 may be performed by the segmentation module 2320.

In some embodiments, a fat mask and a thoracic-abdominal mask may be obtained by segmenting the target structure set (e.g., a second target structure set of an intra-operative image without contrast) from the medical image. Further, the fat-free mask may be obtained based on the fat mask and the thoracic-abdominal mask. In some embodiments, a thoracic-abdominal fat-free mask may be obtained by subtracting the fat mask from the thoracic-abdominal mask. In some embodiments, since the target organ is located in a thoracic-abdominal region, the target organ mask may also be located in a thoracic-abdominal mask region, and the target organ mask may be included in a region of the fat-free mask obtained based on the thoracic-abdominal mask and the fat mask. In some embodiments, the target organ mask may not be included in the region of the fat-free mask obtained based on the thoracic-abdominal mask and the fat mask. For example, a thoracic-abdominal target removal mask may be obtained by subtracting the target organ mask from the thoracic-abdominal mask, and then the fat-free mask may be obtained based on the thoracic-abdominal target removal mask and the fat mask. In some embodiments, the obtaining the fat-free mask by segmenting the target structure set from the medical image may be a segmentation process in the fast segmentation mode (also referred to as a fast segmentation process).

In some embodiments, a processed fat-free mask may be obtained by fusing the fat-free mask and the target organ mask. In some embodiments, the fat-free mask and the target organ mask may be fused using an erosion operation and an expansion operation.

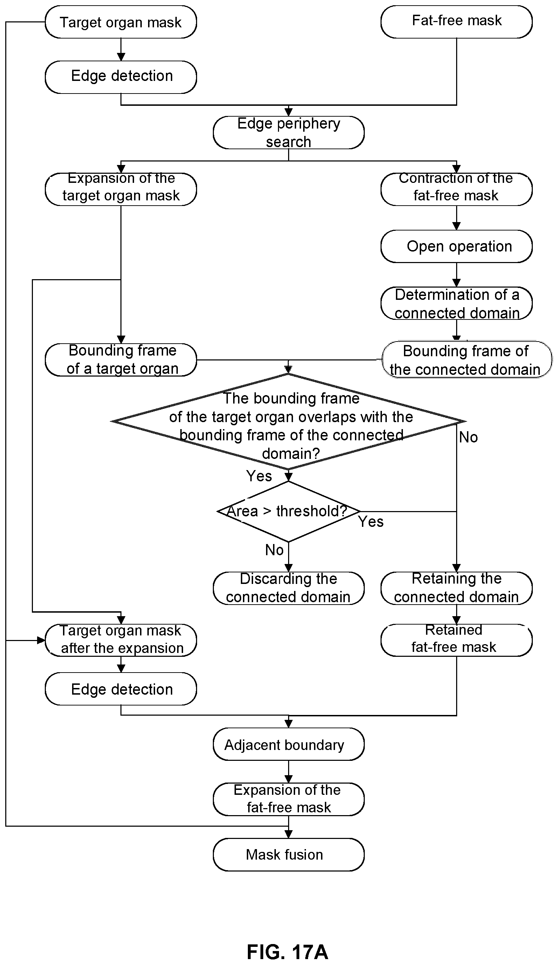

In some embodiments, when the fat-free mask and the target organ mask are fused, in order to ensure that the fat-free mask does not deform, reduce false positive regions, avoid over-segmentation of the target organ mask, and avoid the fat-free mask attaching to the surface of the target organ mask, the fat-free mask and the target organ mask may be fused using a mask fusion algorithm to obtain the processed fat-free mask. More descriptions may be found in FIG. 17A-FIG. 20 and related descriptions thereof.

FIG. 17A is a flowchart illustrating an exemplary mask fusion algorithm according to some embodiments of the present disclosure. The mask fusion algorithm shown in FIG. 17A may be implemented according to the process described in the operation 1620, the operation 1630, and the operation 1640.

In 1620, a first intersection between a target organ mask and the fat-free mask within a first preset range may be determined, and regions of the target organ mask and the fat-free mask may be adjusted based on the first intersection to obtain an adjusted fat-free mask. In some embodiments, the operation 1620 may be performed by the result determination module 2330.

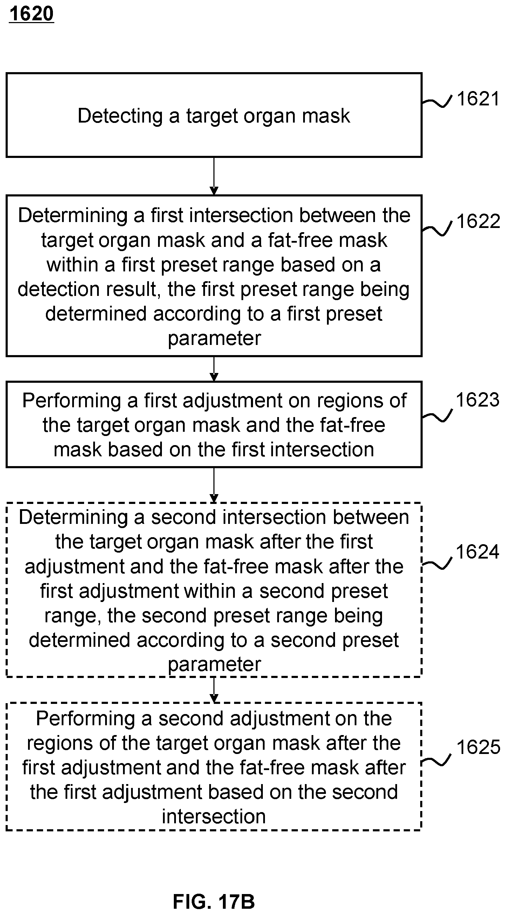

The first preset range refers to a detection range of an element mask (e.g., a target organ mask, and a fat-free mask). In some embodiments, the preset range may include an edge range of the target organ mask. The edge range of the target organ mask may be a mask region that is connected to a boundary of the target organ mask. For example, the target organ mask may be located within a region range of the fat-free mask, and the edge range of the target organ mask may be a partial region of the fat-free mask that is connected to the boundary of the target organ mask. In the region of the preset range, the regions of the target organ mask and the fat-free mask may be adjusted. The adjustment of the mask region may include expansion and contraction of the mask region. The first intersection refers to a mask value of an overlapping portion of the target organ mask and the fat-free mask within the preset range. FIG. 17B is a flowchart illustrating an exemplary process of determining a first intersection and adjusting an element mask based on the first intersection according to some embodiments of the present disclosure. In some embodiments, referring to FIG. 17A and FIG. 17B, the operation 1620 may include the following sub-operations.

In 1621, the target organ mask may be detected.

Detecting the target organ mask may include an edge detection of the target organ mask. For example, a boundary of the target organ mask may be detected. The boundary of the target organ mask may include edge point information of the target organ mask. In some embodiments, edge points of the target organ mask may be determined by detecting an edge of the target organ mask (e.g., the edge detection of the target organ mask is performed in FIG. 17A). Specifically, pixel points of the target organ mask may be detected. If it is detected that a pixel point within a region range (e.g., a preset range) of the target organ mask has null values in any of six directions in a 3D space, the pixel point may be determined as an edge point. This is because the target organ mask is a geometric mask composed of the same pixel point values, and in the case of the edge, an adjacent region of the pixel points of the edge has a null value.

In 1622, the first intersection between the target organ mask and the fat-free mask within the first preset range may be determined based on a detection result, the first preset range being determined according to a first preset parameter.

The first preset range may be a first detection range of the element mask (e.g., the target organ mask, and the fat-free mask). Pixel points within the first preset range of the target organ mask may be detected to determine whether the pixel points are edge points. In some embodiments, whether the edge points of the target organ mask have a fat-free mask value within the first preset range may be determined. If the edge points of the target organ mask have the fat-free mask value within the first preset range, the fat-free mask value may be recorded. Similarly, whether all edge points of the target organ mask have the fat-free mask value within the first preset range may be determined, and all fat-free mask values that meet the condition may be recorded. In some embodiments, the first intersection of the edge points of the target organ mask and the fat-free mask within the first preset range may be determined based on the recorded fat-free mask values. The first intersection may be a region formed by all the recorded fat-free mask values and the edge points of the target organ mask corresponding to each fat-free mask value. In some embodiments, the region of the first intersection may be understood as that in this region, the fat-free mask is attached to the surface of the target organ mask. In some embodiments, the first intersection between the edge of the target organ mask and the fat-free mask within the first preset range may be determined by performing an "edge periphery search" on the edge of the target organ mask and the fat-free mask as shown in FIG. 17A.

In some embodiments, the first preset range may be determined according to the first preset parameter. In some embodiments, an adjustment amplitude of the target organ mask based on the first intersection may be adjusted by adjusting the first preset parameter. For example, the first preset parameter may be set to be relatively small (e.g., 3 pixels), which can make the adjustment amplitude of the region of the target organ mask relatively small. In some embodiments, the first preset parameter may be a constant value obtained by experimental observation. Merely by way of example, the first preset parameter may be 3 to 4 pixels. In some embodiments, the first preset parameter may be reasonably set according to historical data and/or big data. For example, in some embodiments, the first preset ranges may be determined under different first preset parameters in the big data and/or the historical data, and ranges of the first intersections obtained under the first preset ranges may be collected. The ranges of the first intersections may affect the subsequent adjustment amplitude of the target organ mask and the fat-free mask, and then affect the region of the fat-free mask after the final adjustment. Therefore, the regions of the fat-free mask after the final adjustment corresponding to different first preset parameters may be determined, so as to determine a range of a first preset parameter corresponding to a region with a more accurate and clear fat-free mask. In addition, the range of the first preset parameter may also be optimized and adjusted according to feedback of a doctor, such that a more accurate fat-free mask may be obtained under the range of the adjusted first preset parameter. In some embodiments, when the first preset parameter for adjusting a certain mask (referred to as a first target mask) is determined, a plurality of masks (referred to as first comparison masks) having similar conditions (e.g., a similar adjustment amplitude) as the first target mask may be searched and determined from the big data and/or the historical data, then a similarity between the first target mask and each of the first comparison masks may be determined to select a first comparison mask with a relatively high similarity to the first target mask, thereby determining the first preset parameter of the first target mask according to the first preset parameter of the first comparison mask. In some embodiments, the first preset parameter may be determined based on the big data and/or the historical data using a machine learning model. In some embodiments, an input of the machine learning model may include a first adjustment parameter. The first adjustment parameter may include one or more of the adjustment amplitude of the first intersection, the adjustment amplitude of the target organ mask, the adjustment amplitude of the fat-free mask, etc. An output of the machine learning model may include the first preset parameter. The machine learning model may be obtained by training based on the big data and/or the historical data. For example, the machine learning model may be obtained by training an initial machine learning model using the big data and/or the historical data as training samples. In some embodiments, a value of the first preset parameter may also be adjusted according to patient information (e.g., gender, age, a physical condition, etc.) and different organs of the same patient to adapt to a clinical situation.

In 1623, a first adjustment may be performed on the regions of the target organ mask and the fat-free mask based on the first intersection.

The first adjustment performed on the regions of the target organ mask may include expanding the regions of the target organ mask. The first adjustment performed on the regions of the fat-free mask may include contracting the region of the fat-free mask. In some embodiments, the regions of the target organ mask and the fat-free mask may be adjusted with an edge of the first intersection as a boundary. In some embodiments, the regions of the target organ mask may be expanded with the edge of the first intersection formed by all recorded fat-free mask values as the boundary; the regions of the fat-free mask may be contracted with the edge of the first intersection formed by edge points of the target organ mask corresponding to each recorded fat-free mask value as the boundary. In some embodiments, the first adjustment performed on the regions of the target organ mask and the fat-free mask based on the first intersection may be local. For example, only an edge region of the target organ mask may be expanded, and an edge region of the fat-free mask may be contracted, while other regions of a target organ mask body and a fat-free mask body may maintain constant.

In some embodiments, by expanding the regions of the target organ mask and contracting the regions of the fat-free mask, the connection between most of false positive regions and a mask body (e.g., the fat-free mask body, and the target organ mask body) may be disconnected, while the over-segmentation of the fat-free mask may be normalized.

In 1624, a second intersection between the target organ mask after the first adjustment and the fat-free mask after the first adjustment within a second preset range may be determined, the second preset range being determined according to a second preset parameter.

The second preset range may be a second detection range of an element mask (e.g., the target organ mask, and the fat-free mask). In some embodiments, whether an edge point of the target organ mask after the first adjustment (e.g., the expansion) has a fat-free mask value after the first adjustment (e.g., the contraction) within the second preset range may be determined. If an edge point of the target organ mask after the first adjustment (e.g., the expansion) has a fat-free mask value after the first adjustment (e.g., the contraction) within the second preset range, the fat-free mask value may be recorded. Similarly, whether all edge points of the target organ mask after the first adjustment (e.g., the expansion) have fat-free mask values after the first adjustment (e.g., the contraction) within the second preset range may be determined, and all the fat-free mask values that meet the condition may be recorded. Further, the second intersection between the target organ mask after the first adjustment and the fat-free mask after the first adjustment within the second preset range may be determined based on the recorded fat-free mask value. The second intersection may be all the recorded fat-free mask values, and a region formed by the edge points of the target organ mask after the first adjustment corresponding to each recorded fat-free mask value.

In some embodiments, the second preset range may be determined according to the second preset parameter. In some embodiments, an adjustment amplitude of the fat-free mask based on the second intersection may be adjusted by adjusting the second preset parameter. For example, the second preset parameter may be set to be small (e.g., 1 pixel), which can make the adjustment amplitude of the region of the fat-free mask small. In some embodiments, the second preset parameter may be less than or equal to the first preset parameter. Correspondingly, the second preset range may be less than or equal to the first preset range. In some embodiments, the second preset parameter may be a constant value obtained by experimental observation. Merely by way of example, the second preset parameter may be 1-2 pixels. In some embodiments, the second preset parameter may also be reasonably set according to machine learning and/or big data. For example, in some embodiments, the second preset ranges determined under different second preset parameters in the big data and/or the historical data, and the ranges of the second intersections obtained under the second preset ranges may be collected, and the ranges of the second intersections may affect the subsequent adjustment amplitude of the target organ mask and the fat-free mask, which affects the region of the fat-free mask after the final adjustment. Therefore, the regions of the fat-free mask after the final adjustment corresponding to different second preset parameters may be determined, such that the range of the second preset parameter corresponding to a region with a more accurate fat-free mask may be determined. In addition, the range of the second preset parameter may also be optimized and adjusted according to the feedback of the doctor, such that a more accurate fat-free mask may be obtained within the range of the adjusted second preset parameter. In some embodiments, when the second preset parameter for adjusting a certain mask (referred to as a second target mask) is determined, a plurality of masks (referred to as second comparison masks) having similar conditions (e.g., a similar adjustment amplitude) as the second target mask may be searched and determined from the big data and/or the historical data, then a similarity between the second target mask and each of the second comparison masks may be determined to select a second comparison mask with a relatively high similarity to the second target mask, thereby determining the second preset parameter of the second target mask according to the second preset parameter of the second comparison mask. In some embodiments, the second preset parameter may be determined based on the big data and/or the historical data using the machine learning model. In some embodiments, an input of the machine learning model may include a second adjustment parameter. The second adjustment parameter may include one or more of the adjustment amplitude of the second intersection, the adjustment amplitude of the target organ mask, the adjustment amplitude of the fat-free mask, etc. An output of the machine learning model may include the second preset parameter. The machine learning model may be obtained by training based on the big data and/or the historical data. For example, the machine learning model may be obtained by training an initial machine learning model using the big data and/or the historical data as training samples. In some embodiments, a value of the second preset parameter may also be adjusted according to the patient information (e.g., the gender, the age, the physical condition, etc.) and different organs of the same patient to adapt to the clinical situation.

In 1625, a second adjustment may be performed on the regions of the target organ mask after the first adjustment and the fat-free mask after the first adjustment based on the second intersection.

The second adjustment performed on the regions of the target organ mask after the first adjustment may be a second expansion of the regions of the target organ mask after the first adjustment. The second adjustment of the regions of the fat-free mask after the first adjustment may include a second contraction of the regions of the fat-free mask after the first adjustment. In some embodiments, the second adjustment may be performed on the regions of the target organ mask after the first adjustment and the fat-free mask after the first adjustment with an edge of the second intersection as a boundary. In some embodiments, the second expansion may be performed on the regions of the target organ mask after the expansion with an edge of the second intersection formed by all recorded fat-free mask values as a boundary; the second contraction may be performed on the regions of the fat-free mask after the contraction with an edge of the second intersection formed by edge points of the target organ mask after the expansion corresponding to each recorded fat-free mask value as the boundary. In some embodiments, the second adjustment performed on the regions of the target organ mask after the first adjustment and the fat-free mask after the first adjustment based on the second intersection may be global. For example, the second expansion may be performed on the target organ mask after the expansion, and the second contraction may be performed on the fat-free mask after the first contraction. It can be understood that a global adjustment of the element mask (e.g., the target organ mask, and the fat-free mask) may change a main region of the element mask. In some embodiments, the operations described in sub-operations 1624 and 1625 may be regarded as the "open operation" in FIG. 17A. By performing the second expansion on the region of the target organ mask after the first expansion, and performing the second contraction on the fat-free mask after the first contraction, the connection between the rest false positive regions in the sub-operation 1623 and the mask body (e.g., the fat-free mask body, and the target organ mask body) may be disconnected.

In some embodiments, the adjustment amplitude of the mask area may affect other structural tissues in the mask. For example, when the region of the fat-free mask is contracted, the blood vessels in the fat-free mask may be affected. If the region of the fat-free mask contracts to a large extent, it may cause the blood vessels in the fat-free mask to be missing after the contraction. Therefore, the adjustment amplitude of the target organ mask and the fat-free mask area may be controlled by reasonably setting the first preset parameter and the second preset parameter, thereby reducing the impact on other structural tissues in the mask. Merely by way of example, the first preset parameter may be set to 3 pixels and the second preset parameter may be set to 1 pixel, which ensures that the adjustment amplitude of the regions of the target organ mask and the fat-free mask is small, thereby reducing the impact on other structural tissues (e.g., the blood vessels) in the mask, and disconnecting the false positive regions from the mask body and reducing noise regions.

In 1630, a connected domain processing may be performed on the adjusted fat-free mask. In some embodiments, the operation 1630 may be performed by the result determination module 2330.

In some embodiments, after the first adjustment and/or the second adjustment are performed on the regions of the target organ mask and the fat-free mask (e.g., the first expansion and/or the second expansion may be performed on the region of the target organ mask, and the first contraction and/or the second contraction may be performed on the region of the fat-free mask), the connection between the false positive regions and the mask body (e.g., the fat-free mask body, and the target organ mask body) may be disconnected, but more noise regions may appear between the fat-free mask and the target organ mask, which requires a further processing to remove the noise regions.

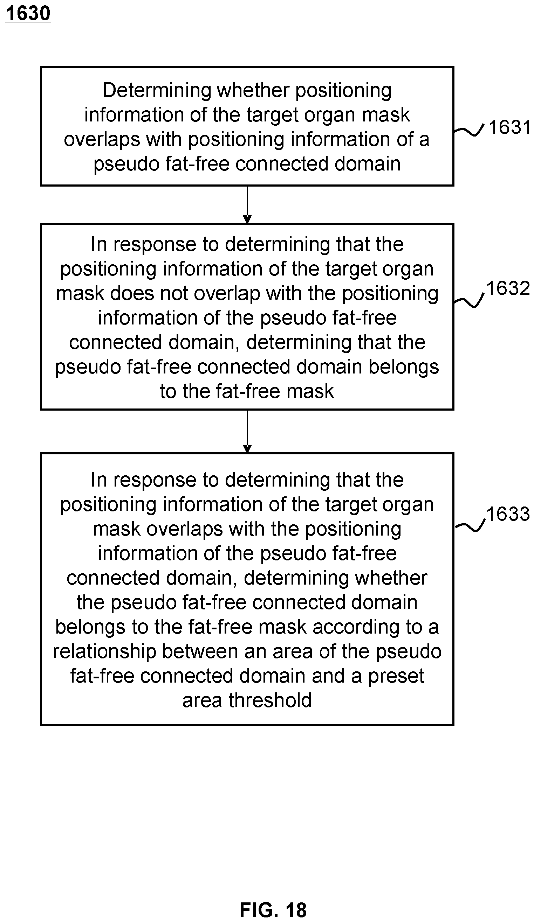

In some embodiments, a connected domain around the adjusted fat-free mask and the adjusted target organ mask may be determined. If the connected domain is a valid connected domain, the connected domain may be retained; if the connected domain is not a valid connected domain, the connected domain may be discarded to remove the noise region. The connected domain, i.e., a connected region, generally refers to an image region composed of foreground pixels with the same pixel value and adjacent positions in the image. FIG. 18 is a flowchart illustrating an exemplary process of performing a connected domain processing on a fat-free mask according to some embodiments of the present disclosure. It can be understood that the fat-free mask in FIG. 18 refers to a fat-free mask that is adjusted, such as a fat-free mask after contraction, and a target organ mask in FIG. 18 refers to a target organ mask that is adjusted, such as a target organ mask after expansion. In some embodiments, referring to FIG. 17A and FIG. 18, the operation 1630 may include the following sub-operations.

In 1631, whether positioning information of the target organ mask overlaps with positioning information of a pseudo fat-free connected domain may be determined.

In 1632, in response to determining that the positioning information of the target organ mask does not overlap with the positioning information of the pseudo fat-free connected domain, it is determined that the pseudo fat-free connected domain belongs to the fat-free mask.

In 1633, in response to determining that the positioning information of the target organ mask overlaps with the positioning information of the pseudo fat-free connected domain, whether the pseudo fat-free connected domain belongs to the fat-free mask may be determined according to a relationship between an area of the pseudo fat-free connected domain and a preset area threshold.

The pseudo fat-free connected domain refers to a connected domain within a peripheral range (e.g., a preset range) of the target organ mask. In some embodiments, the region of the fat-free mask may not be an integrally connected domain, but a region composed of a plurality of connected domains that are not connected with each other. Part of the plurality of connected domains that are not connected with each other may be distributed within the peripheral range of the region of the target organ mask, which are referred to as the pseudo fat-free connected domains.

The positioning information may include position information of a bounding rectangle (i.e., the "bounding frame" in FIG. 17A) of the element mask, such as coordinate information of border lines of the bounding rectangle. In some embodiments, the bounding rectangle of the element mask may cover a positioning region of the corresponding element. In some embodiments, the bounding rectangle may be displayed in the medical image in the form of a bounding rectangle frame. In a 3D space, the bounding rectangle may frame the element mask in the form of a bounding cube. In some embodiments, the bounding rectangle may be a bounding rectangle frame with respect to the element mask constructed based on bottom edges (e.g., the bottom edges of the connected regions in six directions in the 3D space) of the connected regions in the element in various directions.

In some embodiments, for each pseudo fat-free connected domain, whether its positioning information overlaps with the positioning information of the target organ mask may be determined based on the positioning information (e.g., the bounding frame) of the pseudo fat-free connected domain. In some embodiments, in response to determining that the bounding frame of the target organ mask does not overlap with the bounding frame of the pseudo fat-free connected domain, it can be considered that the pseudo fat-free connected domain is not a noise region. In this case, the pseudo fat-free connected domain may belong to the fat-free mask. In response to determining that the bounding frame of the target organ mask overlaps with the bounding frame of the pseudo fat-free connected domain, the pseudo fat-free connected domain can be considered to be a noise region. In this case, whether the pseudo fat-free connected domain belongs to the fat-free mask may be determined based on the relationship between the area of the pseudo fat-free connected domain and the preset area threshold. It can be understood that the area of the pseudo fat-free connected domain refers to an area of a single connected domain. In some embodiments, when the area of the pseudo fat-free connected domain is greater than the preset area threshold, the pseudo fat-free connected domain may belong to the fat-free mask; when the area of the pseudo fat-free connected domain is less than or equal to the preset area threshold, the pseudo fat-free connected domain may not belong to the fat-free mask. In some embodiments, the connected domain not belonging to the fat-free mask may be determined as a false positive region (i.e., the noise region). In some embodiments, the preset area threshold may be a constant value obtained through experimental observation. In some embodiments, when the medical image is a 3D medical image, the preset area threshold may be in a range of 10,000 voxels-1,000,000 voxels. Merely by way of example, the preset area threshold may be 20,000 voxels, 50,000 voxels, 100,000 voxels, etc. In some embodiments, when the medical image is a 2D medical image, the preset area threshold may be in a range of 100 pixels-10,000 pixels. Merely by way of example, the preset area threshold may be 300 pixels, 1000 pixels, 5000 pixels, etc. In some embodiments, the preset area threshold may be reasonably set according to the size of the target organ mask and/or the fat-free mask. In some embodiments, the preset area threshold may also be reasonably set according to machine learning and/or big data, which is not limited here.

In some embodiments, the pseudo fat-free connected domain may be processed according to a determination result. The determination result may include a retaining identifier and/or a discarding identifier. The retaining identifier represents pseudo fat-free connected domains belonging to the fat-free mask. The pseudo fat-free connected domains belonging to the fat-free mask may be retained. The discarding identifier represents pseudo fat-free connected domains not belonging to the fat-free mask. The pseudo fat-free connected domains not belonging to the fat-free mask may be discarded. In some embodiments, the determination result of the pseudo fat-free connected domain may be manually adjusted. For example, a doctor may adjust the identifier of the pseudo fat-free connected domain based on clinical experience. Merely by way of example, if the doctor determines that a pseudo fat-free connected domain belonging to the fat-free mask is a false positive region based on the clinical experience, the pseudo fat-free connected domain may be discarded through manual adjustment.

In some embodiments, when the target organ mask overlaps with the pseudo fat-free connected domain and an area of an overlapping region between the target organ mask overlaps and the pseudo fat-free connected domain is very small, the relationship between the area of the pseudo fat-free connected domain and the preset area threshold may be ignored and the pseudo fat-free connected domain may be directly discarded.

It should be noted that the target organ mask described in FIG. 18 refers to the target organ mask after the expansion, and correspondingly, the bounding frame of the target organ mask refers to the bounding frame of the target organ mask after the expansion.

By performing the connected domain processing on the fat-free mask, the main region of the fat-free mask and the valid connected region (e.g., a connected domain with an area greater than the preset area threshold) around the target organ mask may be retained, and the noise region (e.g., a connected region with an area less than or equal to the preset area threshold) may be removed to obtain the retained fat-free mask.

In 1640, a processed fat-free mask may be obtained based on a fat-free mask after the connected domain processing. In some embodiments, the operation 1640 may be performed by the result determination module 2330.

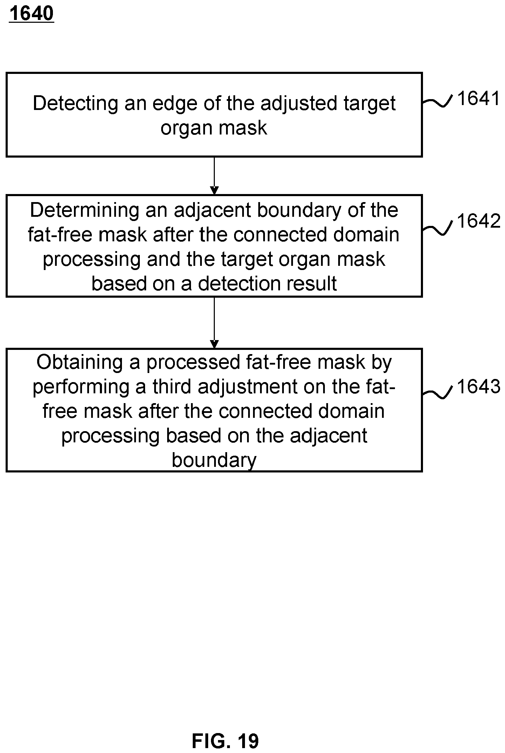

In some embodiments, after the fat-free mask is processed through the operations 1620 and 1630, the connection between the fat-free mask and the target organ mask may be disconnected and the noise region may be removed, but what is obtained at this time may be an adjusted (e.g., contracted) fat-free mask. Accordingly, in order to ensure that the main region of the fat-free mask restores to an original shape, a third adjustment may be performed on the region of the fat-free mask after the connected domain processing to obtain the processed fat-free mask. FIG. 19 is a flowchart illustrating an exemplary process of obtaining a processed fat-free mask based on a fat-free mask after the connected domain processing according to some embodiments of the present disclosure. Referring to FIG. 17A and FIG. 19, the operation 1640 may include the following sub-operations.

In 1641, an edge of the adjusted target organ mask may be determined.

In 1642, an adjacent boundary of the fat-free mask after the connected domain processing and the target organ mask (or the adjusted target organ mask) may be determined based on a detection result.

In 1643, the processed fat-free mask may be obtained by performing a third adjustment on the fat-free mask after the connected domain processing based on the adjacent boundary.

The adjusted target organ mask described in FIG. 19 refers to a target organ mask after the first adjustment and/or the second adjustment, and can also be referred to as an expanded target organ mask; the fat-free mask after the connected domain processing refers to a fat-free mask after the first adjustment and/or the second adjustment, and the connected domain processing. In some embodiments, when the expanded target organ mask is detected, if a pixel point within the range of the expanded target organ mask has a null value in any of the six directions in the 3D space, the pixel point may be determined to be an edge point. More descriptions regarding edge detection may be found above, which are not repeated here.

In some embodiments, the detection result may include edge point information of the expanded target organ mask. The adjacent boundary may be a boundary between the fat-free mask after the connected domain processing and the expanded target organ mask. In some embodiments, the adjacent boundary may be determined according to a detection result of the expanded target organ mask and a detection result of an initial target organ mask (e.g., a target organ mask that is not adjusted). In some embodiments, the adjacent boundary may be determined by determining a difference value between the detection result (e.g., the edge point information) of the expanded target organ mask and the detection result of the initial target organ mask. The processed fat-free mask (also referred to as a non-intervention region mask) may be obtained by performing the third adjustment on the fat-free mask after the connected domain processing based on the adjacent boundary. In some embodiments, the third adjustment performed on the fat-free mask after the connected domain processing may include expanding the fat-free mask after the connected domain processing. The third adjustment performed on the fat-free mask after the connected domain processing may also be referred to as a restoration process of the fat-free mask. The processed fat-free mask may be obtained by expanding the fat-free mask after the connected domain processing. The processed fat-free mask may be a fat-free mask having the same shape as an initial fat-free mask (e.g., a fat-free mask that is not adjusted) and not connected with the initial target organ mask and having no noise region.

The processed fat-free mask may be obtained by fusing the fat-free mask and the target organ mask.

Compared with the morphological erosion operation and the expansion operation, the processed fat-free mask obtained by the mask fusion algorithm can ensure that the processed fat-free mask does not deform, such that the false positive regions are reduced, and over-segmentation of the target organ mask can be avoided, thereby obtaining a more accurate processed fat-free mask. In some embodiments, the processed fat-free mask obtained by the mask fusion algorithm, the initial target organ mask, and the blood vessel mask in the target organ may be used as a fast segmentation result.

It should be noted that the thoracic-abdominal mask and the target organ mask may be fused using the mask fusion algorithm. That is, the thoracic-abdominal mask may be directly fused with the target organ mask without the fat-free operation. The specific processing manner of mask fusion is described above, which is not repeated here.

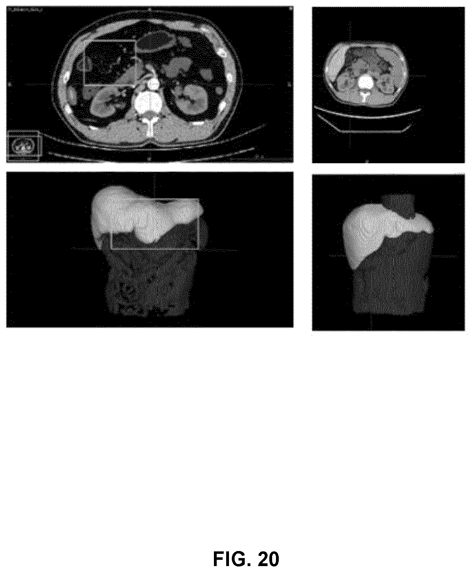

FIG. 20 is a comparison diagram illustrating an exemplary effect of fusing a fat-free mask with a target organ mask according to some embodiments of the present disclosure. As shown in FIG. 20, the upper and lower left sides show a cross-sectional medical image and a stereoscopic medical image of a fusion effect without using a mask fusion algorithm, and the upper and lower right sides show a cross-sectional medical image and a stereoscopic medical image of the fusion effect using the mask fusion algorithm. It can be seen that, in the result of fusing the fat-free mask with the target organ mask based on the mask fusion algorithm, the false positive regions framed by the square frames in the left images are removed, and deformation of the fat-free masks (compare the lower left and lower right images) is avoided.

It should be noted that the above description of the process 1600 is only for example and explanation, and does not limit the scope of application of present disclosure. For those skilled in the art, various modifications and changes can be made to the process 1600 under the guidance of the present disclosure. However, these modifications and changes are still within the scope of protection of the present disclosure.

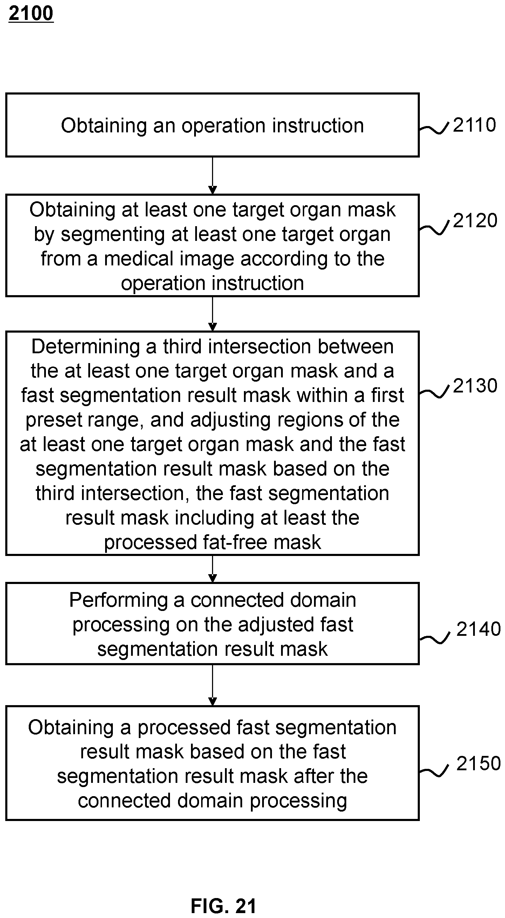

In some embodiments, in order to improve the accuracy of the segmentation results of the medical image, the target organ mask may be obtained by segmenting a target organ from the medical image, and the target organ mask may be fused with the fast segmentation result mask. In some embodiments, the obtaining the target organ mask by segmenting the target organ from the medical image may be a segmentation process (also referred to as a precise segmentation process) in a precise segmentation mode. FIG. 21 is a flowchart illustrating an exemplary process of combining a precise segmentation with a fast segmentation according to some embodiments of the present disclosure. As shown in FIG. 21, a process 2100 may include the following operations.

In 2110, an operation instruction may be obtained. In some embodiments, the operation 2110 may be performed by the obtaining module 2310.

The operation instruction may include a mode instruction and a data instruction. The mode instruction refers to an input instruction on whether the precise segmentation is required, i.e., whether a target organ needs to be segmented from a medical image. The target organ may be an important organ/tissue in the medical image, such as a blood vessel, a bone, etc. In some embodiments, when the mode instruction is that the precise segmentation is not required, a result output by a medical image processing system is a fast segmentation result. In some embodiments, when the mode instruction is that precise segmentation is required, the result output by the medical image processing system may be a fusion result of the fast segmentation result and a precise segmentation result. In some embodiments, when the target organ needs to be segmented from the medical image, the organ that needs to be segmented (i.e., the target organ) may be selected through the data instruction.

In 2120, at least one target organ mask may be obtained by segmenting at least one target organ from the medical image according to the operation instruction. In some embodiments, the operation 2120 may be performed by the segmentation module 2320.

In some embodiments, the at least one target organ may be selected, and the at least one target organ mask may be obtained by segmenting the selected target organ by the segmentation module 2320. In some embodiments, the manner for segmenting the at least one target organ may be the same as the method for the fast segmentation. For example, the manner for segmenting the at least one target organ may include a threshold segmentation manner, a regional growth manner, or a level set manner, or a manner based on a deep learning convolutional network. More descriptions regarding the manner may be found in the manner for the fast segmentation, which are not repeated here.

It is understood that the main difference between the fast segmentation and the precise segmentation is that the organs and/or tissues segmented are different and the segmentation results obtained are different. In the precise segmentation, all organs may be segmented from the medical image to obtain a single organ mask, and the segmentation image may be more detailed and accurate. In the fast segmentation, only the one or more non-interventional regions may be segmented from the medical image as a whole to obtain one or more non-interventional region masks, and the segmentation efficiency may be higher. Therefore, the fast segmentation and the precise segmentation may be combined to improve the accuracy of the segmentation result while ensuring a high segmentation efficiency. For example, the fast segmentation and the precise segmentation may be combined, the segmentation may be performed in the fast segmentation mode to obtain the one or more non-interventional region masks, and a selective precise segmentation may be performed on a specific organ in the precise segmentation mode to obtain a segmentation mask of the specific organ. Then the segmentation mask of the specific organ of the selective segmentation may be fused with the one or more non-interventional region masks (i.e., the segmentation result of the precise segmentation may be displayed on the fast segmentation result).

In 2130, a third intersection between the at least one target organ mask and a fast segmentation result mask within a first preset range may be determined, and regions of the at least one target organ mask and the fast segmentation result mask may be adjusted based on the third intersection, the fast segmentation result mask including at least the processed fat-free mask. In some embodiments, the operation 2130 may be performed by the result determination module 2330.

In some embodiments, the process for determining the third intersection may be similar to the process for determining the first intersection. For example, in some embodiments, the at least one target organ mask may be detected; the third intersection between the at least one target organ mask and the fast segmentation result mask within the first preset range may be determined based on a detection result; and a first adjustment may be performed on the regions of the at least one target organ mask and the fast segmentation result mask based on the third intersection. For example, the regions of the at least one target organ mask may be expanded based on the third intersection, and the regions of the fast segmentation result mask may be contracted. The first adjustment performed on the regions of the at least one target organ mask and the fast segmentation result mask may be local. In some embodiments, similar to the first intersection, the third intersection may be a region composed of all recorded fast segmentation result mask values and edge points of the target organ mask corresponding to each fast segmentation result mask value. More descriptions regarding the process for determining the third intersection may be found above (e.g., the operation 1610 in FIG. 16 and the related descriptions thereof).

After the at least one target organ mask is expanded and the fast segmentation result mask is contracted, the connection between a false positive region and a mask body (e.g., a fast segmentation result mask body, and a target organ mask body) may be disconnected.

In 2140, a connected domain processing may be performed on the adjusted fast segmentation result mask. In some embodiments, the operation 2140 may be performed by the result determination module 2330.

In some embodiments, the connected domain processing performed on the adjusted fast segmentation result mask may be substantially the same as the process of performing the connected domain processing on the adjusted fat-free mask described in the operation 1630 in FIG. 16. In some embodiments, performing the connected domain processing on the adjusted fast segmentation result mask may include: determining whether positioning information of the at least one target organ mask overlaps with positioning information of a pseudo fast segmentation result connected domain; in response to determining that the positioning information of the at least one target organ mask does not overlap with the positioning information of the pseudo fast segmentation result connected domain, the pseudo fast segmentation result connected domain may belong to the fast segmentation result mask; in response to determining that the positioning information of the at least one target organ mask overlaps with the positioning information of the pseudo fast segmentation result connected domain belongs to the fast segmentation result mask according to a relationship between an area of the pseudo fast segmentation result connected domain and a preset area threshold. If the area of the pseudo fast segmentation result connected domain is greater than the preset area threshold, the pseudo fast segmentation result connected domain may belong to the fast segmentation result mask, and the pseudo fast segmentation result connected domain may be retained. When the area of the pseudo fast segmentation result connected domain is less than or equal to the preset area threshold, the pseudo fast segmentation result connected domain may be a false positive region (i.e., a noise region), and the pseudo fast segmentation result connected domain may be discarded. In some embodiments, in response to determining that the target organ mask overlaps with the pseudo fast segmentation result connected domain, and an area of an overlapping region between the target organ mask and the pseudo fast segmentation result connected domain is very small, the relationship between the area of the pseudo fast segmentation result connected domain and the preset area threshold may be ignored, and the pseudo fast segmentation result connected domain may be discarded. The pseudo fast segmentation result connected domain refers to a connected domain within a peripheral range (e.g., the first preset range) of the at least one target organ mask. In some embodiments, the pseudo fast segmentation result connected domain may not be an integrally connected domain, but a region composed of a plurality of connected domains that are not connected with each other. Part of the plurality of connected domains that are not connected with each other may be distributed within the peripheral range of the region of the target organ mask, which are referred to as the pseudo fast segmentation result connected domains.

By performing the connected domain processing on the fast segmentation result mask, the region of the fast segmentation result mask body and a valid connected domain (i.e., a connected domain with an area greater than the preset area threshold) around the target organ mask can be retained, and the noise region (i.e., a connected domain with an area less than or equal to the preset area threshold) can be removed.

In 2150, a processed fast segmentation result mask may be obtained based on the fast segmentation result mask after the connected domain processing. In some embodiments, the operation 2150 may be performed by the result determination module 2330.

For the fast segmentation result mask after the connected domain processing, obtaining the processed fast segmentation result mask may include: detecting the target organ mask after the expansion (i.e., the adjusted target organ mask); determining an adjacent boundary of the fast segmentation result mask after the connected domain processing and the target organ mask (or the adjusted target organ mask) based on a detection result; obtaining the processed fast segmentation result mask by adjusting the fast segmentation result mask after the connected domain processing based on the adjacent boundary. For example, the fast segmentation result mask after the connected domain processing may be expanded (i.e., restored) such that the fast segmentation result mask after the connected domain processing may be restored to an original form.

It is understood that a mask fusion manner (i.e., a mask fusion manner of a precise segmentation result and a fast segmentation result) of the target organ mask and the fast segmentation result mask may be substantially the same as the mask fusion manner of the target organ mask and the fat-free mask in FIG. 16. The difference is that when the target organ mask is fused with the fast segmentation result mask, one time of contraction may be performed on the fast segmentation result mask, eliminating the process of "open operation." This is because when the target organ mask is fused with the fast segmentation result mask, the fast segmentation result mask may not be allowed to change, while the "open operation" may cause the fast segmentation result mask to change. In some embodiments, in order to ensure that the fast segmentation result mask does not change, in the process of determining the third intersection, the first preset parameter may be set to 4 pixels. More descriptions regarding the mask fusion between the target organ mask and the fast segmentation result mask may be found in FIG. 16 and the related descriptions thereof, which are not repeated here.

FIG. 22 is a comparison diagram illustrating exemplary effects of fusing a precise segmentation result mask with a fast segmentation result mask according to some embodiments of the present disclosure. As shown in FIG. 22, the upper and lower left sides show a cross-sectional medical image and a stereoscopic medical image of a fusion effect without using a mask fusion algorithm, and the upper and lower right sides show a cross-sectional medical image and a stereoscopic medical image of a fusion effect using the mask fusion algorithm. Referring to the upper left image, there is a connection between peripheries of two target organ (i.e., two organs framed by square frames) masks and the fast segmentation result mask. By comparing the upper left image with the upper right image, it can be seen that the result of fusing the fast segmentation result mask with the precise segmentation result mask (e.g., the target organ mask) based on the mask fusion algorithm shows that the connection between the target organ mask and the fast segmentation result mask can be disconnected. Referring to the lower left image, two target organ (i.e., two organs framed by a square frame) masks are covered by the fast segmentation result mask. By comparing the lower left image with the lower right image, it can be seen that the result of fusing the fast segmentation result mask with the precise segmentation result mask (e.g., the target organ mask) based on the mask fusion algorithm shows that the target organ mask can be prevented from being covered by the fast segmentation result mask.

It should be noted that the above description of the process 2100 is only for example and explanation, and does not limit the scope of application of the present disclosure. For those skilled in the art, various modifications and changes can be made to the process 2100 under the guidance of the present disclosure. However, these modifications and changes are still within the scope of protection of the present disclosure.

FIG. 23 is a block diagram illustrating an exemplary a system for an image-assisted interventional surgery according to some embodiments of the present disclosure. A system 2300 for an image-assisted interventional surgery may include an obtaining module 2310, a segmentation module 2320, and a result determination module 2330. In some embodiments, the obtaining module 2310, the segmentation module 2320, and the result determination module 2330 may be implemented in the system 100 for the image-assisted interventional surgery shown in FIG. 1, such as in the medical scanning device 110.

The obtaining module 2310 may be configured to obtain a medical image. For example, the obtaining module 2310 may be configured to obtain a pre-operative image with contrasts and an intra-operative image without contrast. In some embodiments, the obtaining module 2310 may be further configured to obtain an operation instruction. The segmentation module 2320 may be configured to segment a target structure set from the medical image. For example, the segmentation module 2320 may be configured to segment a first target structure set from the pre-operative image with contrast and a second target structure set from the intra-operative image without contrast. The result determination module 2330 may be configured to determine a result structure set relating to one or more non-intervention regions based on a segmentation result of the target structure set. For example, the result determination module 2330 may be configured to determine a spatial position of a third target structure set during the surgery based on a segmentation image (e.g., a first segmentation image and a second segmentation image). As another example, the result determination module 2330 may be configured to determine a fat-free mask based on an element mask.

It should be noted that more technical details regarding the obtaining module 2310, the segmentation module 2320, and the result determination module 2330 implementing the corresponding processes or functions to achieve the image-assisted interventional surgery may be found in the related descriptions of the method for the image-assisted interventional surgery described in any embodiment shown in FIGs. 1-22, which are not repeated here.

The above description of the system 2300 for the image-assisted interventional surgery is for illustrative purposes only and is not intended to limit the scope of the present disclosure. For those having ordinary skills in the art, various forms and details of improvements and changes may be made to the application of the above methods and systems without departing from the principle of the present disclosure. However, these improvements and changes will not deviate from the scope of the present disclosure. In some embodiments, the system 2300 for the image-assisted interventional surgery may include one or more other modules. For example, the system 2300 for the image-assisted interventional surgery may include a storage module configured to store data generated by the one or more modules of the system 2300 for the image-assisted interventional surgery.

Some embodiments of the present disclosure further provide a device for an image-assisted interventional surgery. The device may include a processor. The processor may be configured to implement the method for the image-assisted interventional surgery described in any embodiment. More descriptions may be found in FIGs. 1-22, which are not repeated here. In some embodiments, the device for the image-assisted interventional surgery may further include a display device. The display device may be configured to display a segmentation result of the method for the image-assisted interventional surgery implemented by the processor. The display device may be further configured to display trigger mode options. The trigger mode options may include a fast segmentation mode and a precise segmentation mode. An operator may select an appropriate planning mode via the trigger mode options displayed by the display device.

Some embodiments of the present disclosure further provide a non-transitory computer-readable storage medium. The non-transitory computer-readable storage medium may store computer instructions that, when read by a computer, may direct the computer to execute the method for the image-assisted interventional surgery described in any embodiment. More descriptions may be found in FIGs. 1-22, which are not repeated here.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure and are within the spirit and scope of the exemplary embodiments of this disclosure.

Moreover, certain terminology has been used to describe embodiments of the present disclosure. For example, the terms "one embodiment," "an embodiment," and "some embodiments" mean that a particular feature, structure, or feature described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures, or features may be combined as suitable in one or more embodiments of the present disclosure.

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose and that the appended claims are not limited to the disclosed embodiments, but, on the contrary, are intended to cover modifications and equivalent arrangements that are within the spirit and scope of the disclosed embodiments. For example, although the implementation of various parts described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. This method of disclosure, however, is not to be interpreted as reflecting an intention that the claimed subject matter requires more features than are expressly recited in each claim. Rather, claimed subject matter may lie in less than all features of a single foregoing disclosed embodiment.

In some embodiments, numbers describing the number of ingredients and attributes are used. It should be understood that such numbers used for the description of the embodiments use the modifier "about," "approximately," or "substantially" in some examples. Unless otherwise stated, "about," "approximately," or "substantially" indicates that the number is allowed to vary by $\pm 20\%$. Correspondingly, in some embodiments, the numerical parameters used in the description and claims are approximate values, and the approximate values may be changed according to the required features of individual embodiments. In some embodiments, the numerical parameters should consider the prescribed effective digits and adopt the method of general digit retention. Although the numerical ranges and parameters used to confirm the breadth of the range in some embodiments of the present disclosure are approximate values, in specific embodiments, settings of such numerical values are as accurate as possible within a feasible range.

Finally, it should be understood that the embodiments described in the present disclosure are only used to illustrate the principles of the embodiments of the present disclosure. Other variations may also fall within the scope of the present disclosure. Therefore, as an example and not a limitation, alternative configurations of the embodiments of the present disclosure may be regarded as consistent with the teaching of the present disclosure. Accordingly, the embodiments of the present disclosure are not limited to the embodiments introduced and described in the present disclosure explicitly.

**Claims**

1. A method for an image-assisted interventional surgery, comprising:

   obtaining a medical image;
   segmenting a target structure set from the medical image; and
   determining a result structure set relating to one or more non-interventional regions based on a segmentation result of the target structure set.

2. The method of claim 1, wherein the medical image includes a pre-operative image with contrast and an intra-operative image without contrast, the target structure set includes a first target structure set of the pre-operative image with contrast and a second target structure set of the intra-operative image without contrast, and the result structure set includes a third intra-operative target structure set;

   the segmenting a target structure set from the medical image includes:

      obtaining a first segmentation image of the first target structure set by segmenting the first target structure set from the pre-operative image with contrast; and
      obtaining a second segmentation image of the second target structure set by segmenting the second target structure set from the intra-operative image without contrast; the first target structure set and the second target structure set having an intersection; and

   the determining a result structure set relating to one or more non-interventional regions based on a segmentation result of the target structure set includes:

determining a spatial position of the third intra-operative target structure set by registering the first segmentation image with the second segmentation image, at least one element of the third target structure set being included in the first target structure set, and at least one element of the third target structure set being not included in the second target structure set.

3. The method of claim 2, further comprising:

obtaining a planning mode of the interventional surgery, wherein the planning mode includes a fast segmentation mode and a precise segmentation mode; and
segmenting a fourth target structure set from the intra-operative image without contrast according to the planning mode.

4. The method of claim 3, wherein in the fast segmentation mode, the fourth target structure set includes the one or more non-interventional regions.

5. The method of claim 4, wherein in the precise segmentation mode, the fourth target structure set includes one or more preset important organs.

6. The method of claim 5, wherein a ratio of a total volume of the one or more preset important organs to a total volume of the one or more non-interventional regions of the fourth target structure set is less than a preset efficiency factor m.

7. The method of claim 6, wherein the preset efficiency factor m is set based on a type of the interventional surgery.

8. The method of any one of claims 1-7, wherein the segmentation includes:

performing a coarse segmentation on at least one element of the target structure set in the medical image;
obtaining a mask of the at least one element;
determining positioning information of the mask; and
performing a precise segmentation on the at least one element based on the positioning information of the mask.

9. The method of claim 2, wherein the registering the first segmentation image with the second segmentation image includes:

determining a registration deformation field by registering the first segmentation image with the second segmentation image; and
determining, based on the registration deformation field and a spatial position of at least part of the at least one element of the first target structure set in the pre-operative image with contrast, a spatial position of the at least one element during the surgery.

10. The method of claim 9, wherein the determining a registration deformation field includes:

determining a first preliminary deformation field based on a registration result corresponding to the at least one element;
determining a second preliminary deformation field of a full image based on the first preliminary deformation field;
determining a registration map of a moving image by deforming the moving image based on the second preliminary deformation field;
obtaining a third preliminary deformation field by registering a region within a first grayscale difference range in the registration map of the moving image with a region within the first grayscale difference range of a fixed image;
determining a fourth preliminary deformation field of the full image based on the third preliminary deformation field; and
obtaining a final registered registration map by registering a region of the registration map based on the fourth preliminary deformation field, the region being within a second grayscale difference range with respect to the fixed image.

11. The method of claim 1, wherein the result structure set includes a fat-free mask;

the performing a segmentation on a target structure set in the medical image includes:
obtaining the fat-free mask by segmenting the target structure set from the medical image; and
the determining a result structure set relating to one or more non-interventional regions based on a segmentation result of the target structure set includes:
determining a first intersection between a target organ mask and the fat-free mask within a first preset range, and adjusting regions of the target organ mask and the fat-free mask based on the first intersection to obtain an adjusted fat-free mask.

12. The method of claim 11, further comprising:

performing a connected domain processing on the adjusted fat-free mask; and
obtaining a processed fat-free mask based on a fat-free mask after the connected domain processing.

13. The method of claim 12, wherein the determining a first intersection between a target organ mask and the fat-free mask within a preset range, and adjusting regions of the target organ mask and the fat-free mask based on the first intersection includes:

> detecting the target organ mask;
> determining the first intersection between the target organ mask and the fat-free mask within the first preset range based on a detection result, wherein the first preset range is determined according to a first preset parameter; and
> performing a first adjustment on the regions of the target organ mask and the fat-free mask based on the first intersection.

14. The method of claim 13, further comprising:

> determining a second intersection between the target organ mask after the first adjustment and the fat-free mask after the first adjustment within a second preset range, wherein the second preset range is determined according to a second preset parameter; and
> performing a second adjustment on the regions of the target organ mask after the first adjustment and the fat-free mask after the first adjustment based on the second intersection.

15. The method of claim 14, wherein the second preset parameter is less than or equal to the first preset parameter, and at least one of the first preset parameter or the second preset parameter is obtained based on big data or artificial intelligence (AI).

16. The method of claim 12, wherein the performing a connected domain processing on the adjusted fat-free mask includes:

> determining whether positioning information of the target organ mask overlaps with positioning information of a pseudo fat-free connected domain;
> in response to determining that the positioning information of the target organ mask does not overlap with the positioning information of the pseudo fat-free connected domain, determining that the pseudo fat-free connected domain belongs to the fat-free mask; or
> in response to determining that the positioning information of the target organ mask overlaps with the positioning information of the pseudo fat-free connected domain, determining whether the pseudo fat-free connected domain belongs to the fat-free mask according to a relationship between an area of the pseudo fat-free connected domain and a preset area threshold.

17. The method of claim 16, wherein the determining whether the pseudo fat-free connected domain belongs to the fat-free mask according to a relationship between an area of the pseudo fat-free connected domain and a preset area threshold includes:

> in response to determining that the area of the pseudo fat-free connected domain is greater than the preset area threshold, determining that the pseudo fat-free connected domain belongs to the fat-free mask; or
> in response to determining that the area of the pseudo fat-free connected domain is less than or equal to the preset area threshold, determining that the pseudo fat-free connected domain does not belong to the fat-free mask.

18. The method of claim 16, further comprising:
make the pseudo fat-free connected domain with at least one of a retaining identifier or a discarding identifier, wherein the retaining identifier represents pseudo fat-free connected domains belonging to the fat-free mask, and the discarding identifier represents pseudo fat-free connected domains not belonging to the fat-free mask.

19. The method of claim 12, wherein the obtaining a processed fat-free mask based on the fat-free mask after the connected domain processing includes:

> detecting the adjusted target organ mask;
> determining an adjacent boundary of the fat-free mask after the connected domain processing and the target organ mask based on a detection result; and
> obtaining the processed fat-free mask by performing a third adjustment on the fat-free mask after the connected domain processing based on the adjacent boundary.

20. The method of claim 12, further comprising:

> obtaining an operation instruction;
> obtaining at least one target organ mask by segmenting at least one target organ in the medical image according to the operation instruction;
> determining a third intersection between the at least one target organ mask and a fast segmentation result mask within a first preset range, and adjusting regions of the at least one target organ mask and the fast segmentation result mask based on the third intersection, wherein the fast segmentation result mask includes at least the processed fat-free mask;

performing a connected domain processing on the adjusted fast segmentation result mask; and obtaining a processed fast segmentation result mask based on the fast segmentation result mask after the connected domain processing.

21. A system for an image-assisted interventional surgery, comprising:

an obtaining module configured to obtain a medical image;
a segmentation module configured to segment a target structure set from the medical image; and
a result determination module configured to determine a result structure set relating to one or more non-interventional regions based on a segmentation result of the target structure set.

22. A non-transitory computer-readable storage medium, comprising computer instructions that, when read by a computer, direct the computer to implement the method of any one of claims 1-20.

23. A device for an image-assisted interventional surgery, comprising a processor, wherein the processor is configured to implement the method of any one of claims 1-20.

24. The device of claim 23, further comprising a display device, wherein the display device is configured to display a segmentation result of the method implemented by the processor, and the display device is further configured to display trigger mode options, the trigger mode options including a fast segmentation mode and a precise segmentation mode.

<u>100</u>

FIG. 1

**200A**

```
┌─────────────────────────────────────────┐
│                                         │  ～／210A
│      Obtaining a medical image          │
│                                         │
└─────────────────────────────────────────┘
                    │
                    ▼
┌─────────────────────────────────────────┐
│  Segmenting a target structure set      │  ～／220A
│  from the medical image                 │
└─────────────────────────────────────────┘
                    │
                    ▼
┌─────────────────────────────────────────┐
│  Determining a result structure set     │
│  relating to one or more                │  ～／230A
│  non-interventional regions based       │
│  on a segmentation result of the        │
│  target structure set                   │
└─────────────────────────────────────────┘
```

**FIG. 2A**

**200B**

Obtaining a first segmentation image of a first target structure set by segmenting the first target structure set from a pre-operative image with contrast ~/210B

Obtaining a second segmentation image of a second target structure set by segmenting the second target structure set from an intra-operative image without contrast ~/220B

Determining a spatial position of a third target structure set during the surgery by registering a first segmentation image with a second segmentation image ~/230B

**FIG. 2B**

<u>300</u>

```
┌─────────────────────────────────────┐
│  Performing a coarse segmentation    │
│  for at least one element of a       │  ～ 310
│  target structure set in a medical   │
│  image                               │
└─────────────────────────────────────┘
                  │
                  ▼
┌─────────────────────────────────────┐
│  Obtaining a mask of the at least    │  ～ 320
│  one element                         │
└─────────────────────────────────────┘
                  │
                  ▼
┌─────────────────────────────────────┐
│  Determining positioning information │  ～ 330
│  of the mask                         │
└─────────────────────────────────────┘
                  │
                  ▼
┌─────────────────────────────────────┐
│  Performing a precise segmentation   │
│  on the at least one element based   │  ～ 340
│  on the positioning information of   │
│  the mask                            │
└─────────────────────────────────────┘
```

**FIG. 3**

**330**

Determining a count of connected domains ⟶ 331

In response to determining that the count of the connected domains is ≥ 2, determining an area of connected domains that meet a preset condition ⟶ 332

In response to determining that a ratio of an area of the largest connected domain of a plurality of connected domains to a total area of the connected domains is greater than a first threshold M, determining that the largest connected domain meets the preset condition ⟶ 333

Determining retained connected domains that include at least the largest connected domain ⟶ 334

Determining positioning information of an element mask based on the retained connected domains ⟶ 335

**FIG. 4**

Element mask

↓

Connected domain analysis

| Count of connected domains = 2 | Count of connected domains = 0 | Count of connected domains = 1 | Count of connected domains > 3 |

Obtaining connected domains A and B

Mask being null

Retained connected domain

Obtaining connected Domains A, B, and C

Determining an area of the connected domains

Determining an area of the connected domains

Determining based on a first threshold M

Determining based on a first threshold M or a second threshold N

Retained connected domain A

Retained connected domains A and B

Retained connected domain A

Determining again based on the first threshold M or the second threshold N

Retained connected domains A and B

Retained connected domains A, B, and C

↓

Outputting the retained connected domains

**FIG. 5**

FIG. 6

**340**

Performing a preliminary precise segmentation on at least one element — 341

Determining whether positioning information of an element mask is accurate — 342

Yes

No

In response to determining that a determination result is inaccurate, obtaining accurate positioning information based on an adaptive sliding window — 343a

In response to determining that the determination result is accurate, outputting a preliminary precise segmentation result — 343b

**FIG. 7**

FIG. 8

FIG. 9

**FIG. 10A**

**FIG. 10B**

**FIG. 10C**

B1-3

C

B

A

B1

First direction

Second direction

**FIG. 10D**

B1-1

B

A

B1-2

B1

First direction

Second direction

**FIG. 10E**

**FIG. 11**

**230B**

Determining a registration deformation field by registering a first segmentation image with a second segmentation image  ~ 231B

Determining a spatial position of the at least one element during the surgery based on the registration deformation field and a spatial position of at least part of the at least one element of a first target structure set in a pre-operative image with contrast  ~ 232B

**FIG. 12**

## 231B

Determining a first preliminary deformation field based on a registration result corresponding to at least one element  ~/2311B

Determining a second preliminary deformation field of a full image based on the first preliminary deformation field  ~/2312B

Determining a registration map of a moving image by deforming the moving image based on the second preliminary deformation field  ~/2313B

Obtaining a third preliminary deformation field by registering a region within a first grayscale difference range in the registration map of the moving image with a region within the first grayscale difference range of a fixed image  ~/2314B

Determining a fourth preliminary deformation field of the full image based on the third preliminary deformation field  ~/2315B

Obtaining a final registered registration map by registering a region of the registration map based on the fourth preliminary deformation field, the region being within a second grayscale difference range with respect to the fixed image  ~/2316B

FIG. 13

FIG. 14

Pre-operative image with contrast    Intra-operative image without contrast

Segmentation

Organ contour A                    Organ contour B

**FIG. 15**

**1600**

Obtaining a fat-free mask by segmenting a target structure set from a medical image ⟍⟋1610

↓

Determining a first intersection between a target organ mask and the fat-free mask within a first preset range, and adjusting regions of the target organ mask and the fat-free mask based on the first intersection to obtain an adjusted fat-free mask ⟍⟋1620

↓

Performing a connected domain processing on the adjusted fat-free mask ⟍⟋1630

↓

Obtaining a processed fat-free mask based on a fat-free mask after the connected domain processing ⟍⟋1640

**FIG. 16**

FIG. 17A

**1620**

Detecting a target organ mask 〜 1621

↓

Determining a first intersection between the target organ mask and a fat-free mask within a first preset range based on a detection result, the first preset range being determined according to a first preset parameter 〜 1622

↓

Performing a first adjustment on regions of the target organ mask and the fat-free mask based on the first intersection 〜 1623

↓

Determining a second intersection between the target organ mask after the first adjustment and the fat-free mask after the first adjustment within a second preset range, the second preset range being determined according to a second preset parameter 〜 1624

↓

Performing a second adjustment on the regions of the target organ mask after the first adjustment and the fat-free mask after the first adjustment based on the second intersection 〜 1625

**FIG. 17B**

<u>1630</u>

Determining whether positioning information of the target organ mask overlaps with positioning information of a pseudo fat-free connected domain ∿ 1631

In response to determining that the positioning information of the target organ mask does not overlap with the positioning information of the pseudo fat-free connected domain, determining that the pseudo fat-free connected domain belongs to the fat-free mask ∿ 1632

In response to determining that the positioning information of the target organ mask overlaps with the positioning information of the pseudo fat-free connected domain, determining whether the pseudo fat-free connected domain belongs to the fat-free mask according to a relationship between an area of the pseudo fat-free connected domain and a preset area threshold ∿ 1633

**FIG. 18**

<u>1640</u>

```
┌─────────────────────────────────────┐
│   Detecting an edge of the adjusted  │  ∿ 1641
│          target organ mask           │
└─────────────────────────────────────┘
                  │
                  ▼
┌─────────────────────────────────────┐
│  Determining an adjacent boundary of │  ∿ 1642
│  the fat-free mask after the connected│
│   domain processing and the target   │
│   organ mask based on a detection    │
│                result                │
└─────────────────────────────────────┘
                  │
                  ▼
┌─────────────────────────────────────┐
│  Obtaining a processed fat-free mask │  ∿ 1643
│  by performing a third adjustment on │
│  the fat-free mask after the connected│
│   domain processing based on the     │
│           adjacent boundary          │
└─────────────────────────────────────┘
```

**FIG. 19**

**FIG. 20**

<u>2100</u>

```
┌─────────────────────────────────────┐
│   Obtaining an operation instruction │ ～2110
└─────────────────────────────────────┘
                    │
                    ▼
┌─────────────────────────────────────┐
│  Obtaining at least one target organ │
│  mask by segmenting at least one     │ ～2120
│  target organ from a medical image   │
│  according to the operation          │
│  instruction                         │
└─────────────────────────────────────┘
                    │
                    ▼
┌─────────────────────────────────────┐
│  Determining a third intersection    │
│  between the at least one target     │
│  organ mask and a fast segmentation  │
│  result mask within a first          │ ～2130
│  preset range, and adjusting         │
│  regions of the at least one target  │
│  organ mask and the fast             │
│  segmentation result mask based on   │
│  the third intersection, the fast    │
│  segmentation result mask including   │
│  at least the processed fat-free     │
│  mask                                │
└─────────────────────────────────────┘
                    │
                    ▼
┌─────────────────────────────────────┐
│  Performing a connected domain       │
│  processing on the adjusted fast     │ ～2140
│  segmentation result mask            │
└─────────────────────────────────────┘
                    │
                    ▼
┌─────────────────────────────────────┐
│  Obtaining a processed fast          │
│  segmentation result mask based on   │
│  the fast segmentation result mask   │ ～2150
│  after the connected domain          │
│  processing                          │
└─────────────────────────────────────┘
```

**FIG. 21**

**FIG. 22**

2300

Obtaining module ∿2310

Segmentation module ∿2320

Result determination module ∿2330

**FIG. 23**

# INTERNATIONAL SEARCH REPORT

| International application No. |
| --- |
| **PCT/CN2023/103728** |

**A. CLASSIFICATION OF SUBJECT MATTER**

G06T 7/11(2017.01)i

According to International Patent Classification (IPC) or to both national classification and IPC

**B. FIELDS SEARCHED**

Minimum documentation searched (classification system followed by classification symbols)

IPC:G06T

Documentation searched other than minimum documentation to the extent that such documents are included in the fields searched

Electronic data base consulted during the international search (name of data base and, where practicable, search terms used)

CNTXT, ENTXT, CNKI, IEEE: 拆分, 分割, 分离, 规避, 交集, 介入, 禁区, 配准, 平扫, 去脂, 脂肪, 扫描, 手术, 图像, 掩膜, 影像, 增强, split, separate, elusion, zone, forbid, register, picture, ct, photo, mask, operation, fat, scan, enhance

**C. DOCUMENTS CONSIDERED TO BE RELEVANT**

| Category* | Citation of document, with indication, where appropriate, of the relevant passages | Relevant to claim No. |
| --- | --- | --- |
| X | CN 113516624 A (WUHAN UNITED IMAGING ZHIRONG MEDICAL TECHNOLOGY CO., LTD.) 19 October 2021 (2021-10-19)<br>description, paragraphs [0081]-[0131], and figures 1-20 | 1, 8, 11-24 |
| Y | CN 113516624 A (WUHAN UNITED IMAGING ZHIRONG MEDICAL TECHNOLOGY CO., LTD.) 19 October 2021 (2021-10-19)<br>description, paragraphs [0081]-[0131], and figures 1-20 | 2-7, 9-10 |
| Y | CN 113506331 A (WUHAN UNITED IMAGING ZHIRONG MEDICAL TECHNOLOGY CO., LTD.) 15 October 2021 (2021-10-15)<br>description, paragraphs [0074]-[0093] | 2-7, 9-10 |
| A | CN 112435263 A (RAYCAN TECHNOLOGY CO., LTD. (SUZHOU)) 02 March 2021 (2021-03-02)<br>entire document | 1-24 |
| A | US 2021401392 A1 (GENENTECH, INC.) 30 December 2021 (2021-12-30)<br>entire document | 1-24 |

☐ Further documents are listed in the continuation of Box C. ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **19 September 2023** | **22 September 2023** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT
### Information on patent family members

| International application No. |
| --- |
| **PCT/CN2023/103728** |

| Patent document cited in search report | | | Publication date (day/month/year) | Patent family member(s) | | | Publication date (day/month/year) |
| --- | --- | --- | --- | --- | --- | --- | --- |
| CN | 113516624 | A | 19 October 2021 | None | | | |
| CN | 113506331 | A | 15 October 2021 | None | | | |
| CN | 112435263 | A | 02 March 2021 | None | | | |
| US | 2021401392 | A1 | 30 December 2021 | WO | 2020190821 | A1 | 24 September 2020 |
| | | | | JP | 2022525198 | A | 11 May 2022 |
| | | | | EP | 3939002 | A1 | 19 January 2022 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2022107613241
- CN 202210907258